(19) Europäisches Patentamt
European Patent Office
Office européen des brevets

(11) Publication number: 0 124 216 B1

(12) EUROPEAN PATENT SPECIFICATION

(45) Date of publication of patent specification: 26.10.88

(51) Int. Cl.[4]: C 07 H 17/08, A 61 K 31/70, A 23 K 1/17

(21) Application number: 84301234.5

(22) Date of filing: 27.02.84

# (54) C-20- and C-23-modified macrolide derivatives.

(30) Priority: 28.02.83 US 470833
28.02.83 US 470890

(43) Date of publication of application:
07.11.84 Bulletin 84/45

(45) Publication of the grant of the patent:
26.10.88 Bulletin 88/43

(84) Designated Contracting States:
AT BE CH DE FR GB IT LI LU NL SE

(56) References cited:
EP-A-0 042 250
EP-A-0 052 005
EP-A-0 070 170
EP-A-0 087 921

CHEM. PHARM. BULL., vol. 30, no. 1, 1982, pages 97-110, H. MATSUBARA et al.: "Chemical transformation of tylosin, a 16-membered macrolide, and its structure-activity relationship"

JOURNAL OF ANTIBIOTICS, vol. 35, no. 1, January 1982, pages 113-116, Tokyo, JP. "Syntheses of 23-dialkylamino derivatives of mycaminosyl tylonolide and 4'-deoxymycaminosyl tylonolide effective against gram-negative bacteria"

(73) Proprietor: ELI LILLY AND COMPANY
307, East McCarty Street
Indianapolis Indiana 46285 (US)

(72) Inventor: Debono, Manuel
5257 Hinesley Avenue
Indianapolis Indiana 46208 (US)
Inventor: Kirst, Herbert Andrew
1819, Madison Village Drive Apt. B-6
Indianapolis Indiana 46227 (US)
Inventor: Toth, John Eldon
121 West Wood Street Apt. 5
West Lafayette Indiana 47906 (US)

(74) Representative: Crowther, Terence Roger et al
Erl Wood Manor
Windlesham Surrey GU20 6PH (GB)

(56) References cited:
THE JOURNAL OF ANTIBIOTICS, vol. 34, no. 10, October 1981, pages 1377-1380, Tokyo, JP. "Syntheses of derivatives of 4'-deoxymycyminosyl tylonolide and mycaminosyl tylonolide modified at C-23"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 124 216 B1

**Description**

This invention related to macrolide antibiotics, and more specifically to a novel group of C—20 and C—23 modified derivatives of tylosin and tylosin-like macrolides.

Improved antibiotics are continually in demand. In addition to antibiotics which are useful for treating human diseases, improved antibiotics are also needed in the veterinary field. Increased potency, expanded spectrum of bacterial inhibition, increased *in vivo* efficacy, and improved pharmaceutical properties (such as greater oral absorption, higher blood or tissue concentrations, longer body half life, and more advantageous rate or route of excretion and rate or pattern of metabolism) are some of the goals for improved antibiotics.

Tylosin is a well-known therapeutic agent in the veterinary field. (See, for example, *Tetrahedron Letters* 1970, 2339 and U.S. Patent No. 3,178,341). Tylosin and tylosin-like macrolides have been modified in an attempt to obtain derivatives having improved properties. A large number of derivatives have been made, but improvement in activity has not previously been obtained to the desired degree.

20-Amino derivatives of Tylosin, and like macrolides, are disclosed in EP—A—87921.

C—20- and C—23-modified tylosin derivatives are described in EP—A—52005, and by Matsuhata et al in Chem. Pharm. Bull. 1982 30(1) 97—110.

More specifically this invention relates to C—20- and C—23-modified macrolide derivatives having formula (I);

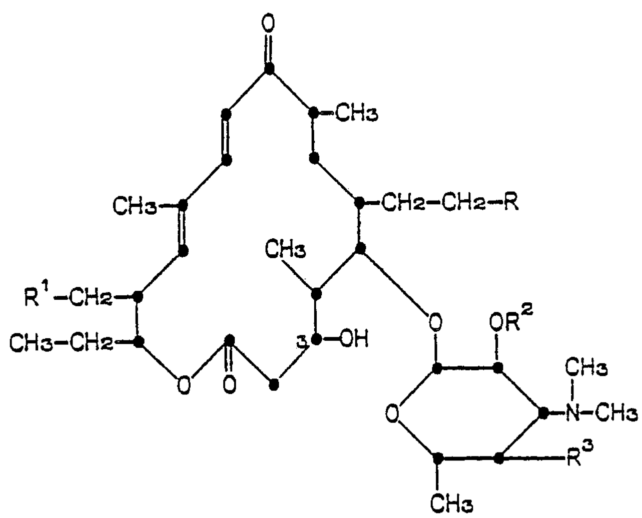

(I)

wherein

R is hydrogen, iodo, bromo, chloro, fluoro, cyano, —$OR^4$, —OAr, —$SR^5$, azido, —$NR^6R^7$, N-phthalimido or $R^9$;

$R^1$ is i) hydrogen or —OH; ii) chloro, fluoro, bromo, iodo, —OAr, —O-tetrahydrofuranyl, —O-tetrahydropyranyl, —$SR^5$, azido, —$NR^6R^7$, or N-phthalimido; or $R^9$;

$R^9$ is i) a monocyclic amino group of the formula —$N(CH_2)_n$ which is optionally substituted at one or more of the carbon atoms by a $C_1$—$C_3$-alkyl, hydroxyl, methoxyl, ethoxyl, —$N(R^8)_2$,

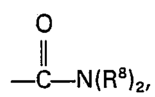

carbomethoxy, carboethoxy, or phenyl group; and n is an integer from 4 through 15; ii) a monocyclic saturated or unsaturated nitrogen-containing heterocyclic ring bonded through the nitrogen atom, said ring having 1) from 5 to 7 ring atoms which include up to 3 additional heteroatoms selected from nitrogen, oxygen and sulfur, and 2) up to 3 substituent groups selected from methyl, ethyl and phenyl; or iii) a bicyclic or tricyclic secondary amino group selected from 1,2,3,4-tetrahydroquinolin-1-yl; decahydroquinolin-1-yl; 1,2,3,4-tetrahydroisoquinolin-2-yl; decahydroisoquinolin-2-yl; indolin-1-yl; isoindolin-2-yl; decahydrocyclohepta[b]pyrrol-1-yl; decahydrocyclohepta[c]pyrrol-2-yl; decahydrocyclopent[c]azepin-2-yl; decahydrocyclopent[d]azepin-3-yl; 2,3,4,5-tetrahydro-1H-2-benzazepin-2-yl; 2,3,4,5-tetrahydro-1H-3-benzazepin-3-yl; azabicycloheptanyl; azabicyclooctanyl; azabicyclononanyl; azabicyclodecanyl or azatricyclodecanyl;

$R^2$ is hydrogen, optionally substituted $C_1$—$C_5$-alkanoyl or optionally substituted benzoyl, phenylacetyl or phenylpropionyl;

$R^3$ is hydrogen, hydroxyl, optionally substituted $C_1$—$C_5$-alkanoyloxy or optionally substituted benzoyloxy, phenylacetoxy or phenylpropionyloxy or

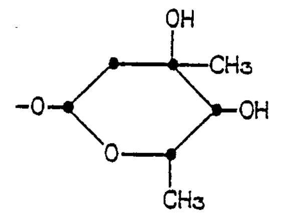 ;

(mycarosyloxy)

$R^4$ is hydrogen, optionally substituted $C_1$—$C_4$-alkyl, cyclohexyl, optionally substituted benzyl, phenethyl or phenoxyethyl;

Ar is i) phenyl, derivatized phenyl, or naphthyl; ii) an optionally substituted heteroaryl group selected from pyridinyl, pyrimidinyl, pyridazinyl, pyrazinyl, triazinyl, indolyl, isoquinolinyl, quinolinyl, quinazolinyl, cinnolinyl, quinoxalinyl, phthalazinyl, benzotriazolyl, benzoxazolyl, benzimidazolyl, carbazolyl, or acridinyl; or iii) optionally substituted $C_1$—$C_5$-alkanoyl; optionally substituted benzoyl, phenylacetyl, phenylpropionyl, phenoxyacetyl or phenylthioacetyl; methanesulfonyl; trifluoromethanesulfonyl; or optionally substituted phenylsulfonyl;

$R^5$ is optionally substituted $C_1$—$C_4$-alkyl; cyclohexyl; optionally substituted phenyl, benzyl or phenethyl; or an optionally substituted heteroaryl group selected from imidazolyl, pyrazolyl, pyridinyl, pyrimidinyl, pyrazinyl, pyridazinyl, triazinyl, triazolyl, tetrazolyl, oxazolyl, isoxazolyl, oxadiazolyl, thiazolyl, isothiazolyl, thiadiazolyl, thienyl and furanyl;

$R^6$ is hydrogen, optionally substituted $C_1$—$C_6$-alkyl, phenyl, benzyl, phenethyl or $C_3$—$C_8$-cycloalkyl;

$R^7$ is an $R^6$ group or optionally substituted $C_1$—$C_5$-alkanoyl, optionally substituted benzoyl, phenylacetyl, phenylpropionyl, phenoxyacetyl or phenylthioacetyl, or alkoxycarbonyl; and

$R^8$ is hydrogen, methyl, ethyl, n-propyl or isopropyl or the $R^8$ groups taken together form a polymethylene moiety such that —$N(R^8)_2$ constitutes a cyclic amino group selected from pyrrolidinyl, piperidinyl, hexahydroazepinyl or octahydroazocinyl;

provided 1) that, when $R^4$ is hydrogen, $R^1$ cannot be hydrogen or —OH; 2) that, when R or $R^1$ is —$NHR^6$ or $R^4$ or $R^8$ is hydrogen, $R^2$ must be hydrogen, $R^3$ must be hydrogen, hydroxyl, or mycarosyloxy and Ar cannot be a type (iii) substituent; 3) that, when $R^2$ is hydrogen, $R^3$ must be hydrogen, hydroxyl or mycarosyloxy; 4) that at least one of R and R′ is $R_9$, 5) that when R′ is H or OH, $R^9$ is not a monocyclic saturated or unsaturated nitrogen-containing heterocyclic ring as defined under ii) and to the salts, particularly the acid addition salts, of these compounds.

The compounds of this invention are useful as antibiotics and/or as intermediates to antibiotics.

Monocyclic saturated or unsaturated nitrogen-containing heterocyclic rings which are bonded through the nitrogen atom and which have from five to seven ring atoms, including up to three additional heteroatoms selected from nitrogen, oxygen and sulfur, include groups such as pyrrolyl, pyrazolyl, imidazolyl, 1,2,4-oxadiazinyl, 1,3,4-thiadiazinyl, 1,2,4-triazolyl, 1H-tetrazolyl, 1,4-diazepinyl, morpholino, thiomorpholino, piperazinyl, thiazolidinyl, oxazolidinyl, and tetrahydro-1,4-thiazin-4-yl. Such rings can have up to three substituents selected from methyl, ethyl and phenyl on appropriate carbon and/or nitrogen ring atom(s).

The term "$C_1$—$C_5$-alkanoyl" as used herein means an acyl moiety derived from a carboxylic acid containing from one to five carbon atoms. In such a moiety, the alkyl group can be straight, branched, or cyclic. When optionally substituted, the alkyl group can bear one to three halo substituents. Halo substituents are selected from Cl, Br and F. Acetyl, chloroacetyl, trichloroacetyl, trifluoroacetyl, propionyl, *n*-butyryl, isobutyryl, *n*-valeryl, and isovaleryl are examples of such groups. The term "$C_1$—$C_5$-alkanoyloxy" refers to the corresponding acyloxy moiety.

The terms "optionally substituted benzoyl, phenylacetyl, phenylpropionyl, phenoxyacetyl or phenylthioacetyl", "optionally substituted benzoyl, phenylacetyl or phenylpropionyl", "optionally substituted benzoyloxy, phenylacetoxy or phenylpropionyloxy", "optionally substituted phenyl, benzyl or phenethyl", "optionally substituted benzyl, phenethyl or phenoxyethyl" and "optionally substituted phenylsulfonyl" mean that the phenyl portion of the moiety is optionally substituted by from one to five halo or methyl groups or by from one to two methoxyl, nitro or hydroxyl groups.

The term "derivatized phenyl" refers to a phenyl group which has from one to five halo, methoxyl or $C_1$—$C_4$-alkyl substituents, or from one to two nitro, amino, methylamino, ethylamino, dimethylamino, diethylamino, $C_4$—$C_{10}$-methyleneamino, azido, hydroxy, hydroxymethyl, aminomethyl, (methylamino)methyl, (ethylamino)methyl, (dimethylamino)methyl, (diethylamino)methyl, ($C_4$—$C_{10}$-methyleneamino)methyl, formyl, acetyl, benzoyl, methoxycarbonyl, ethoxycarbonyl, carboxamido, N-methylcarboxamido, N,N-dimethylcarboxamido, cyano, phenyl, phenoxy or benzyl substituents.

The term "optionally substituted heteroaryl group" as used herein means that the heteroaryl group may have at least one suitable substituent(s) such as a $C_1$—$C_4$-alkyl, halo, methoxy, ethoxy, hydroxy (or the keto tautomer) or phenyl group.

The terms "$C_1$—$C_3$-alkyl", "$C_1$—$C_4$-alkyl" or "$C_1$—$C_6$-alkyl" as used herein mean a straight- or branched-chain alkyl group containing the specified number of carbon atoms. Such groups include methyl,

ethyl, isopropyl, *n*-butyl, *tert*-butyl, *n*-hexyl, and the like. "Optionally substituted" $C_1$—$C_4$-alkyl or $C_1$—$C_6$-alkyl means that the alkyl group contains one or more fluoro or chloro substituents.

"$C_3$—$C_8$-cycloalkyl" refers to a cycloalkyl group containing from three to eight carbon atoms. Examples of such groups are cyclopropyl, cyclohexyl and cyclooctyl.

The term "alkoxycarbonyl" represents a member of a group selected from t-butoxycarbonyl, methoxycarbonyl, ethoxycarbonyl, 2,2,2-trichloroethoxycarbonyl, phenoxycarbonyl and benzyloxycarbonyl.

The term "$C_4$—$C_{10}$-methyleneamino" represents a cyclic amino substituent of the formula $—N(CH_2)_n$ wherein n is an integer from four to ten. Pyrrolidinyl, piperidinyl, and octahydroazocinyl are examples of such groups.

The invention also provides a process for preparing a macrolide of formula (I) by

(a) reducing a starting macrolide of formula (II) wherein Q is formyl and $Q^1$ is $R^1$, provided $R^1$ is not hydroxyl, to give a macrolide of formula (I) wherein R is hydroxyl;

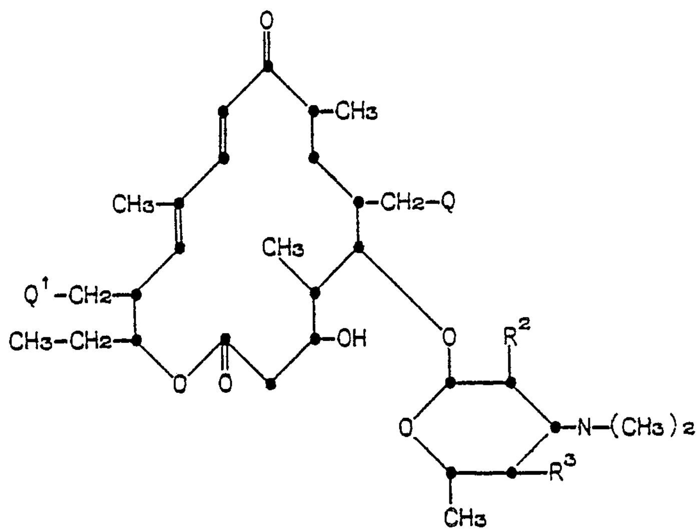

(II)

(b) reacting a starting macrolide of formula (II) wherein Q is formyl and $Q^1$ is $R^1$ with an amine of the formula $HNR^6R^6$ or $HR^9$ in the presence of a reducing agent to give a macrolide of formula (I) wherein R is $NR^6R^6$ or $R^9$, or

(c) reacting a starting macrolide of formula (II) wherein Q is $—CH_2OH$ and $Q^1$ is $R^1$ with diethylazodicarboxylate or dimethylazodicarboxylate, triphenylphosphine, and a reagent selected from

(i) an azide transfer agent, to give a macrolide of formula (I) wherein R is azido,

(ii) phthalimide to give a macrolide of formula (I) wherein R is phthalimido,

(iii) a phenol of formula ArOH to give a macrolide of formula (I) wherein R is —OAr, where Ar is a category 1) or ii) Ar group,

(iv) an alkyl halide or polyhalide to give a macrolide of formula (I) wherein R is Cl, Br, or I, or

(v) a mercaptan of formula $HSR^5$ to give a macrolide of formula (I) wherein R is $SR^5$, or

(vi) a carboxylic or sulfonic acid of the formula ArOH, where Ar is a category (iii) Ar group, to give a macrolide of formula (I) wherein R is OAr,

(d) reacting a starting macrolide of formula (II) wherein Q is $—CH_2OH$ and $Q^1$ is $R^1$ with triphenylphosphine and a halogen source to give a macrolide of formula (I) wherein R is Cl, Br, or I,

(e) reacting a starting material of formula (II) wherein Q is $—CH_2OH$ and $Q^1$ is $R^1$ with an acylating agent derived from a carboxylic or sulfonic acid of formula ArOH, where Ar is a category (iii) Ar group to give a macrolide of formula (I) wherein R is OAr, or

(f) reacting a starting macrolide of formula (II) wherein Q is $—CH_2L$ where L is a leaving group and $Q^1$ is $R^1$ or a leaving group with

(i) an alkali metal azide or halide or a tetraalkylammonium azide or fluoride where alkyl is methyl, ethyl, propyl or butyl, to give a macrolide of formula (I) wherein R is azido, F, Cl, Br, or I, or

(ii) a mercaptide ion of formula $R^5S—$ to give a macrolide of formula (I) wherein R is $R^5S—$, or

(iii) an amine of the formula $NR^6R^6$ or $HR^9$ to give a macrolide of formula (I) wherein R is $NR^6R^6$ or $R^9$, or

(iv) a source of cyanide ion to give a macrolide of formula (I) wherein R is —CN;

(v) an alcohol of the formula $HOR^4$ and a source of silver ion to provide a macrolide of formula (I) wherein R is $OR^4$ where $R^4$ is other than hydrogen

(g) hydrolyzing a macrolide of formula (II) wherein Q is $CH_2R$ and $Q^1$ is

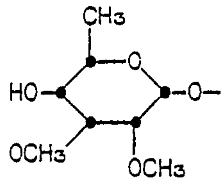 , 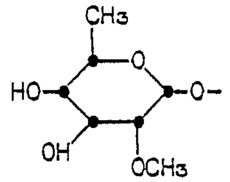 , or 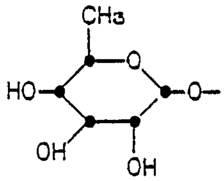

to give a macrolide of formula (I) wherein $R^1$ is hydroxyl, or

(h) removing the hydroxy protecting group from a macrolide of formula (II) wherein Q is $-CH_2R$ and $Q^1$ is protected hydroxy or

(i) reacting a starting macrolide of formula (II) wherein Q is $-CH_2R$ and $Q^1$ is hydroxyl with diethylazodicarboxylate or dimethylazodicarboxylate, triphenylphosphine, and a reagent selected from

(i) an azide transfer agent to give a macroide of formula (I) wherein $R^1$ is azido,

(ii) phthalimide to give a macrolide of formula (I) wherein $R^1$ is phthalimido,

(iii) a phenol of formula ArOH where Ar is a category (i) or (ii) Ar group to give a macrolide of formula (I) wherein $R^1$ is —OAr,

(iv) an alkyl halide or polyhalide to give a macrolide of formula (I) where $R^1$ is Cl, Br, or I, or

(v) a mercaptan of formula $HSR^5$ to give a macrolide of formula (I) wherein R is $SR^5$, or

(vi) a carboxylic or sulfonic acid of the formula ArOH, where Ar is a category (i) Ar group, to give a macrolide of formula (I) wherein $R^1$ is OAr; or

(j) reacting a macrolide of formula (II) wherein Q is $-CH_2R$ and $Q^1$ is hydroxyl with an acylating agent derived from a carboxylic or sulfonic acid of formula ArOH where Ar is a category (iii) Ar group to give a macrolide of formula (I) wherein $R^1$ is OAr or

(k) reacting a macrolide of formula (II) wherein Q is R and $Q^1$ is hydroxyl with triphenylphosphine and a halogenating agent to give a macrolide of formula (I) wherein $R^1$ is Cl, Br or I, or

(l) reacting a macrolide of formula (II) wherein Q is $-CH_2R$ and $Q^1$ is a leaving group with

(i) an alkali metal azide or halide or a tetraalkylammonium azide or fluoride where alkyl is methyl, ethyl, propyl, or methyl to give a macrolide of formula (I) wherein $R^1$ is azido, F, Cl, Br, or I,

(ii) a mercaptide ion of formula $R^5S-$ to give a macroide of formula I wherein $R^1$ is $R^5S-$, or

(iii) an amine of the formula $HNR^6R^6$ or $HR^9$ to give a macrolide of formula (I) wherein $R^1$ is $HR^6R^6$ or $R^9$, or

(m) reducing a macrolide of formula (II) wherein Q is $-CH_2N_3$ and $Q^1$ is $R^1$ or Q is R and $Q^1$ is azido to give a macrolide of formula (I) wherein R is $-CH_2NH_2$ or $R^1$ is amino, or

(n) acylating a macrolide of formula (II) wherein Q is $-CH_2NHR^6$ or $R^1$ is $NHR^6$ to give a macrolide of formula (I) wherein R is $-CH^2NHR^7$ or $R^1$ is $-NR^6R^7$, or

(o) esterifying a macrolide of formula (I), or

(p) salifying a macrolide of formula (I), or

(q) hydrolyzing a macrolide of formula (I) wherein $R^3$ is mycarosyloxy in acid solution at a pH below 4 to give a macrolide of formula (I) wherein $R^3$ is hydroxy, or

(r) deoxygenating a macrolide of formula (I) wherein $R^3$ is hydroxy to give a macrolide of formula (I) wherein $R^3$ is hydrogen.

(s) reacting a macrolide of formula (II) wherein Q is $CH_2I$ and $Q^1$ is $R^1$ with a reducing agent to give a compound of formula (I) wherein R is hydrogen, or

(t) reacting a macrolide of formula (II) wherein Q is $-CH_2-$ sulfonate and $Q^1$ is $R^1$ with a source of iodide ion to give a macrolide of formula (I) wherein R is iodo.

In general, macrolides of formula (I) are prepared by effecting a modification at the C—20 position of a macrolide that has the desired group at the C—23 position, or by effecting a modification at the C—23 position of a macrolide that has the desired group at the C—20 position, or by effecting modifications at the 20- and 23-positions simultaneously. In addition, macrolides of formula (I) may be modified at 2′-, 4′-, 20-, and 23-positions using known methods to produce other macrolides of formula (I).

The following are known macrolides which are useful in preparing the macrolides of this invention: demycinosyltylosin (DMT), 20-dihydro-23-demycinosyltylosin (dihydro-DMT), 23-de(mycinosyloxy)tylosin (DMOT), 20-dihydro-23-de(mycinosyloxy)tylosin (dihydro-DMOT), 5-O-mycaminosyltylonolide (OMT), 20-dihydro-5-O-mycaminosyltylonolide (dihydro-OMT), 23-deoxy-5-O-mycaminosyltylonolide (DOMT), 20-dihydro-23-deoxy-5-O-mycaminosyltylonolide (dihydro-DOMT), 20-dihydro-20-deoxy-23-demycinosyltylosin (DH—DO—DMT) and 20-dihydro-20-deoxy-5-O-mycaminosyltylonolide (DH—DO—OMT).

DMT, dihydro-DMT, DMOT, dihydro-DMOT, DOMT, and dihydro-DOMT are antibiotics described by Richard H. Baltz, Gene M. Wild, and Eugene T. Seno in U.S. Patents 4,321,361 and 4,321,362, both of which issued on March 23, 1982. DH—DO—DMT and DH—DO—OMT are described by Richard H. Baltz, Herbert A. Kirst, Gene H. Wild and Eugene T. Seno in U.S. Patent 4,304,856, which issued December 8, 1981. OMT and dihydro-OMT are described by Marvin Gorman and Robert D. Morin in U.S. Patent 3,459,853, issued on August 5, 1969.

The structures of the starting antibiotics are shown in formulas *2—11*:

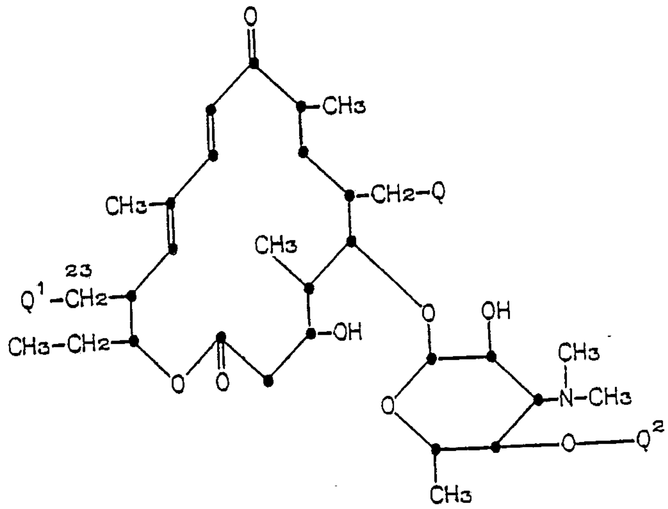

| | Q | $Q^1$ | $Q^2$ |
|---|---|---|---|
| *2* DMT: | —CHO | —OH | mycarosyl |
| *3* dihydro-DMT: | —$CH_2$OH | —OH | " |
| *4* OMT: | —CHO | —OH | H |
| *5* dihydro-OMT: | —$CH_2$OH | —OH | H |
| *6* DMOT: | —CHO | H | mycarosyl |
| *7* dihydro-DMOT: | —$CH_2$OH | H | " |
| *8* DOMT: | —CHO | H | H |
| *9* dihydro-DOMT: | —$CH_2$OH | H | H |
| *10* DH—DO—DMT | —$CH_3$ | —OH | mycarosyl |
| *11* DH—DO—OMT | —$CH_3$ | —OH | H |

Other known macrolides are also useful starting materials, as will be clear from the following discussion.

Methods for modifying the C—20-position

The desired group at the C—20 position can be obtained by using a known starting material that already has the desired group, or by modifying the C—20 position of an available starting material. The C—20 position may be modified before or after the other required modifications, if any, are made in the starting material.

Known macrolides of formula (II) wherein Q is —$CH_2$OH include dihydro-DMT (*3*), dihydro-OMT (*5*), dihydro-DMOT (*7*), and dihydro-DOMT (*9*).

Macrolides of formula (I) or (II) wherein R is hydroxyl or Q is $CH_2$OH can be prepared by reducing a macrolide of formula (II) wherein Q is formyl to provide the corresponding 20-dihydro compound. Chemical reduction can be effected, for example, by treating the macrolide with an approximately stoichiometric amount of sodium borohydride in an alcoholic solvent.

Macrolides of formula (I) or (II) wherein R is OH or Q is $CH_2$OH may be converted to other macrolides of formula (I) by modifying the C—20 hydroxyl group, using any of a variety of known synthetic methods. Several methods are described hereinafter.

Known macrolides of formula (II) wherein Q is methyl include DH—DO—DMT (*10*) and DH—DO—OMT (*11*).

Macrolides of formula (I) or (II) wherein R is hydrogen or Q is methyl can be prepared by reacting a macrolide of formula (II) wherein Q is —$CH_2$I with a reducing agent such as a hydride, for example sodium borohydride in a dipolar aprotic solvent such as dimethyl sulfoxide, dimethylformamide, or sulfolane, or an organotin hydride such as tri-n-butyltin hydride, or a metal, for example powdered zinc, in a nonreactive organic solvent such as toluene or nitromethane.

Macrolides of formula (I) or (II) wherein R is Cl, Br, or I, or Q is —$CH_2$Cl, —$CH_2$Br, or —$CH_2$I are prepared

by reacting a macrolide of formula (II) where Q is —$CH_2OH$ with triphenylphosphine and a halogenating agent in a nonreactive organic solvent such as dichloromethane. Suitable halogenating agents include alkyl halides and polyhalides, such as carbon tetrachloride.

Macrolides of formula (I) or (II) wherein R is Cl, Br, or I, or Q is —$CH_2Cl$, —$CH_2Br$, or —$CH_2I$ are also prepared by reacting a macrolide of formula (II) wherein Q is —$CH_2OH$ with triphenylphosphine, diethylazodicarboxylate or dimethylazodicarboxylate, and an alkyl halide or polyhalide.

The di(alkyl)azodicarboxylate/triphenylphosphine reaction and its various applications are described in O. Mitsunobu, *Synthesis, 1* (1), 1—28 (1981). The reaction generally produces dehydration between an alcohol ROH and an acidic component HX to provide a product RX.

Macrolides of formula (I) or (II) wherein R is F, Cl, Br, or I, or Q is —$CH_2F$, —$CH_2Cl$, —$CH_2Br$, or —$CH_2I$ are also prepared by reacting a macrolide of formula (II) wherein Q is —CHO with an alkali metal or tetra(alkyl)-ammonium halide in a nonreactive organic solvent such as tetrahydrofuran.

Macrolides of formula (I) or (II) wherein R is azido or Q is —$CH_2N_3$ are prepared by reacting a macrolide of formula (II) wherein Q is —CHO with an alkali metal or tetra(alkyl)ammonium azide in a nonreactive organic solvent. Macrolides of formula (I) or (II) wherein R is azido or Q is —$CH_2N_3$ are also prepared by reacting a macrolide of formula (II) wherein Q is —$CH_2OH$ with triphenylphosphine, diethylazodicarboxylate or dimethylazodicarboxylate, and an azide transfer agent, such as diphenylphosphoryl azide, in a nonreactive organic solvent such as tetrahydrofuran.

Macrolides of formula (I) or (II) wherein R is cyano or Q is —$CH_2CN$ are prepared by reacting a macrolide of formula (II) wherein Q is —$CH_2L$, where L is halide or a sulfonic ester group, with a cyanide salt in a non-reactive organic solvent, such as dimethylsulfoxide.

Macrolides of formula (I) or (II) wherein R is $OR^4$ or Q is $CH_2OR^4$ are prepared by reacting a macrolide of formula (II) wherein Q is $CH_2L$ with an alcohol of the formula $HOR^4$, where $R^4$ is other than hydrogen in the presence of a source of silver ion.

Macrolides of formula (I) or (II) wherein R is OAr or Q is $CH_2OAr$ are prepared by reacting a macrolide of formula (II) wherein Q is —$CH_2OH$, with triphenylphosphine, di(alkyl)azodicarboxylate, where alkyl is methyl or ethyl, and a phenol of formula AroH, where Ar is a category i) or ii) Ar group.

Macrolides of formula (I) or (II) wherein R is OAr or Q is $CH_2OAr$ and Ar is optionally substituted benzoyl, phenylacetyl, phenylpropionyl, phenoxyacetyl, or phenylthioacetyl are prepared by reacting a macrolide of formula (II) wherein Q is —$CH_2OH$ with an acylating agent derived from a carboxylic acid of formula ArOH, where Ar is as defined above. Typical acylating agents include anhydrides, acid halides (usually in combination with a base or other acid scavenger), and active esters. Suitable organic solvents include pyridine and triethylamine. Acylation can also be achieved using a mixture of an organic acid and a dehydrating agent such as N,N'-dicyclohexylcarbodiimide.

Macrolides of formula (I) or (II) wherein R is OAr or Q is $CH_2OAr$ and Ar is an acyl group as defined in the previous paragraph are also prepared using the di(alkyl)azodicarboxylate/triphenylphosphine procedure, i.e., by reacting a macrolide of formula (II) wherein Q is —$CH_2OH$ with triphenylphosphine, di(alkyl)azodicarboxylate, and a carboxylic acid of the formula ArOH.

Macrolides of formula (I) or (II) wherein R is OAr or Q is $CH_2OAr$ and Ar is methanesulfonyl, trifluoromethanesulfonyl or optionally substituted phenylsulfonyl are prepared by reacting a macrolide of formula (I) wherein Q is —$CH_2OH$ with an activated derivative, such as the anhydride or acid halide, of a sulfonic acid of the formula ArOH. If the acid halide is used, the reaction is carried out in the presence of a base, usually pyridine.

Macrolides of formula (I) or (II) wherein R is OAr or Q is $CH_2OAr$ and Ar is defined in the previous paragraph are also prepared using the di(alkyl)azodicarboxylate/triphenylphosphine procedure, i.e., by reacting a macrolide of formula (II) wherein Q is —$CH_2OH$ with triphenylphosphine, di(alkyl)azodicarboxylate and a sulfonic acid of the formula ArOH.

Macrolides of formula (I) or (II) wherein R is $SR^5$ or Q is —$CH_2SR^5$ are prepared by reacting a macrolide of formula (II) wherein Q is —$CH_2L$ with mercaptide ion of the formula $R^9S^-$. L may be halide or a sulfonic or sulfonic ester group.

Macrolides of formula (I) or (II) wherein R is $SR^5$ or Q is —$CH_2SR^5$ are also prepared using the di(alkyl)-azodicarboxylate/triphenylphosphine procedure, i.e., by reacting a macrolide of formula (II) wherein Q is —$CH_2OH$ with triphenylphosphine, di(alkyl)azodicarboxylate and a mercaptan of formula $HSR^5$.

Macrolides of formula (I) or (II) wherein R is $NR^6R^7$ or Q is —$CH_2NR^6R^7$ are prepared by reacting a macrolide of formula (II) wherein Q is —$CH_2NHR^6$ with an acylating agent derived from a carboxylic acid of the formula $HOR^7$.

Macrolides of formula (I) or (II) wherein R is phthalimido, or Q is —$CH_2$-phthalimido are prepared by reacting a macrolide of formula (II) wherein Q is —$CH_2OH$ with triphenylphosphine, di(alkyl)azodicarboxylate, and phthalimide.

Compounds of formula (I) or (II) wherein $R^4$ or L is methanesulfonyl, trifluoromethanesulfonyl or optionally substituted phenylsulfonyl, as well as compounds wherein R or L is iodo or bromo, are useful as intermediates for the preparation of additional compounds of this invention via $S_N1$ or $S_N2$ substitution reactions. Suitable reaction conditions for displacing a leaving group by a nucleophile via either an $S_N1$ or $S_N2$ mechanism are well exemplified in the art of nucleophilic substitution reactions.

The formula (I) or (II) compounds wherein R is —$NHR^7$ and $R^7$ is an acyl group are prepared via the 20-

azido derivative ($R = N_3$). The 20-azido derivative is first reduced to the 20-amino derivative ($R = NH_2$); triphenylphosphine in aqueous tetrahydrofuran (THF) is an example of a suitable reducing agent for this purpose. The 20-amino derivative can then be selectively acylated on the amino group, using standard acylation procedures, to give those derivatives wherein $R^7$ is an acyl group.

Macrolides of formula (I) or (II) wherein R is $—N(R^6)_2$ or $R^9$ or Q is $CH_2N(R^6)_2$ or $CH_2R^9$ can be prepared by reductive amination of the C—20 aldehyde group of DMT, OMT, DMOT, and DOMT using one of two methods.

Method 1:

A derivative with a leaving group at C—20 (iodo or triflate), prepared as described *supra*, is reacted with the appropriate amine in a suitable solvent, such as acetonitrile, until the desired 20-modified derivative is formed via displacement of the C—20 leaving group by the nucleophilic amine.

Method 2:

In this method, the aldehyde group of compound *2, 4, 6*, or *8* is reacted directly with the corresponding amine in the presence of a suitable reducing agent in an appropriate solvent until the desired product is formed. Sodium cyanoborohydride is an example of a suitable reducing agent, and anhydrous methanol is a useful solvent for this reaction. The reaction may be carried out under a nitrogen atmosphere, but this is usually not required. With less reactive amines, more forcing conditions for forming the intermediate iminium complex between the macrolide and amine may be required, e.g. heating, use of a drying agent or water scavenger or heating under conditions of azeotropic removal of water in solvents such as benzene or toluene.

Methods for modifying the C—23-position

The desired group at the C—23 position is likewise obtained by using an available starting material that already has the desired group at the C—23 position, or by modifying the C—23 position of an available starting material.

Known macrolides of formula (II) wherein $Q^1$ is hydroxyl include DMT (*2*), dihydro-DMT (*3*), OMT (*4*), dihydro-OMT (*5*), DH—DO—DMT (*10*), and DH—DO—OMT (*11*).

Macrolides of formula (I) or (II) wherein $R^1$ or $Q^1$ is hydroxyl can be prepared by acid hydrolysis of a macrolide of formula (II) wherein $Q^1$ is

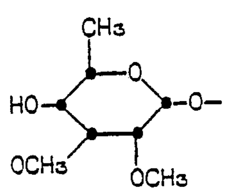 , 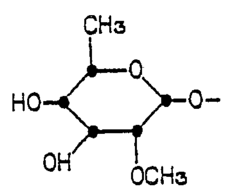 , or 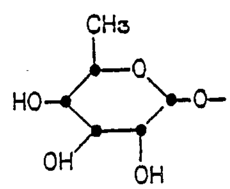

The hydrolysis is carried out at a pH between 1.5 and 2.5, as described in U.S. Patent No. 3,459,853.

Macrolides of formula (I) or (II) wherein $R^1$ or $Q^1$ is hydroxyl can be modified to give other macrolides of formula (I) using the methods described hereinbefore for converting a C—20 hydroxyl group to the desired group. Thus, the DEAD reaction may be used to effect many of the C—23 modifications. Other procedures for modifcation of the C—23 position are described by A. Tanaka et al in *J. Antibiotics 35* (1) 113—116 (1982).

Another process applicable to C—23 modifications is a two-step process in which the 23-hydroxyl group is first converted to a leaving group, and the leaving group is then displaced by a suitable nucleophile.

First, the 23-hydroxyl group is converted to a suitable leaving group, such groups being well known in the art. The triflate anion is a preferred leaving group. With very reactive nucleophiles, however, other leaving groups, such as the mesylate anion, the tosylate anion, iodide or bromide may also be suitable.

The 23—O-triflate is preferably prepared by reaction of the 23—OH intermediate with an activated derivative of trifluoromethanesulfonic acid, such as the anhydride, preferably in the presence of a hindered pyridine derivative such as 2,6-lutidine or s-collidine at a temperature of from −80°C to room temperature. The hydroacyl groups at positions other than 23- may be protected by acetyl groups which can be removed by methanolysis, for instance by refluxing in methanol. Using this procedure, the 23—O-triflate can be prepared without concomitant reactions at the other hydroxyl groups which are present. A similar reaction can be used to prepare the corresponding mesylate or tosylate directly.

When triflate is used as the leaving group, it is not necessary to isolate the intermediate triflate derivative; displacement with the appropriate nucleophile can be carried out *in situ*. When less reactive leaving groups are used, the intermediate is sufficiently stable and may be isolated prior to the displacement reaction if so desired.

The second step in the preparation of the C—23 modified derivatives involves displacement of the

leaving group by the appropriate nucleophile under suitable conditions which are well exemplified in the art of displacement reactions.

When it is desired to prepare compounds of formula (II) in which $Q^1$ is $SR^5$ the most convenient nucleophile is a compound of formula $HSR^5$. For preparation of an azide, the nucleophile is preferably an alkali metal azide such as lithium azide. The pyridinium compound is preferably prepared by reaction with pyridine base. When $Q^1$ is $NR^6R^7$ or $R^9$, the nucleophile is an amine of the formula $HNR^6R^7$ or $HR^9$.

The nucleophilic displacement reaction is preferably conducted at temperatures in the range from −80°C to 100°C, typically room temperature using an inert organic solvent such as a chlorinated hydrocarbon like dichloromethane.

The C—23 derivatives wherein $R^1$ is $—NHR^7$ can be prepared via the 23-azido derivative ($R = N_3$). The 23-azido derivative is first reduced to the 23-amino derivative using a reducing agent specific to azido groups, such as chromous chloride or triphenylphosphine. Aqueous organic solvents such as ethereal solvents, for example tetrahydrofuran (THF) are useful for this purpose. The reduction can be effected at temperatures in the range from 0 to 100°C. The 23-amino derivative can then be selectively acylated using standard acylation procedures, to give those derivatives wherein $R^7$ is an acyl group. As will be appreciated by those skilled in the art the acylation can be effected at temperatures in the range of from −20 to 70°C.

It should be noted that, when the compounds of formulas *3* or *5* are used as starting materials, two primary hydroxyl groups are present which react in a similar manner. The primary hydroxyl group at C—20, however, is usually replaced more rapidly than the hydroxyl group at C—23. Although many of the procedures described supra give mixtures of 20-monosubstituted derivatives and 20,23-disubstituted derivatives, such mixtures can be readily separated by techniques known in the art, such as, for example, chromatography using silica gel as the adsorbent. Formation of C—20-monosubstituted derivatives may be optimized by not carrying the reaction to completion, for example, by using less than two molar equivalents of reactant(s). Conversely, when C—20, C—23-disubstituted derivatives are sought, the reaction should be carried to completion and two molar equivalents or an excess of reactant(s) should be used.

Compounds wherein the substituent $R^1$ differs from the substituent R can be prepared by modifying the hydroxyl group at C—23 before reducing the aldehyde at C—20.

When preparing formula (I) compounds wherein R is hydrogen, compounds *10* and *11* may be used as starting materials and modified at the C—23 hydroxyl group as previously described.

An alternate method for preparing compounds with different substituents at C—20 and C—23 is to modify the C—20 position of a macrolide not having a free C—23 hydroxyl group. One example of this approach is to prepare a C—20-modified derivative of desmycosin, tylosin, macrocin, lactenocin, demethylmacrocin and demethyllactenocin, followed by hydrolysis at the neutral sugar(s), using procedures known in the art (see, for example, U.S. Patent 3,459,853). By this procedure, a 20-modified derivative of OMT can be selectively prepared, which in turn can be modified at the C—23 position, as discussed *supra*.

Use of a protecting group for the hydroxyl group at C—23 of OMT and DMT prior to reduction of the aldehyde also permits selective modification of C—20. Removal of the protecting group after appropriate modification of C—20 yields C—20-modified derivatives having a hydroxyl group at C—23, which may then be modified as outlined previously. Examples of useful protecting groups are ester moieties, such as acetyl and trichloroacetyl, and groups such as tetrahydropyranyl and tetrahydrofuranyl. The tetrahydropyranyl and tetrahydrofuranyl protecting groups are described, for example, by Tanaka et al., *supra*.

The modified derivatives of OMT, DOMT and DH—DO—OMT can also be prepared by acidic hydrolysis of mycarose from the corresponding modified derivatives of DMT, DMOT and DH—DO—DMT, respectively, prepared by the methods previously described. Procedures for the acidic hydrolysis of mycarose from DMT and DMOT to form OMT and DOMT, respectively, are found in U.S. Patents 4,321,361 and 4,321,362. Acidic hydrolysis of DH—DO—DMT to give DH—DO—OMT is described in U.S. Patent 4,304,856.

More specifically, the mycarose sugar can be hydrolytically cleaved at a pH of less than 4, preferably in the range from 0.5 to 2.0, at a temperature in the range of from 0 to 60°C, conveniently at about room temperature. The hydrolysis can be effected using a strong aqueous mineral acid such as hydrochloric or sulfuric acid or a strong organic acid such as *p*-toluenesulfonic acid.

A method of preparing 4′-deoxydesmycosin is described in *J. of Antibiotics 34*, 1381—84 (1981). The process involves (1) treatment of desmycosin with acidic ethanol in accordance with a procedure described in *Antibiot. & Chemoth. 11*, 320—27 (1961), to obtain the corresponding diethylacetal; (2) acylation of the diethylacetal with acetic anhydride in acetonitrile in the absence of external base, in accordance with a procedure described in *J. Org. Chem. 44*, 2050—52 (1979, to obtain the 2′,4′-di-O-acetyl derivative; (3) reacting the 2′,4′-di-O-acetyl derivative with 2,3-dihydrofuran in dichloromethane in the presence of pyridinium p-toluenesulfonate in the manner described in *J. Org. Chem. 42*, 3772—74 (1974) to obtain the 3,4′′-bis(O-tetrahydrofuranyl)derivative; (4) removal of the 2′ and 4′-O-acetyl groups by dissolving the product of step (3) in methanol (50°C, overnight); (5) reacting the product of step (4) with 1.5 mole equivalent of benzenesulfonyl chloride in pyridine at −40°C for 4 hours, to provide the 4′-O-benzenesulfonyl derivative; (6) immediately reacting the 4′-O-benzenesulfonyl derivative with 1.5 equivalent of sodium iodide in methyl ethyl ketone at 180°C for 15 minutes to obtain 4′ iodo derivative; (7) reductively deiodinating the 4′-iodo derivative using tri(n-butyl)stannane in benzene in the presence of 2,2′-azobis-isobutyronitrile at 80°C for 2 hours; and (8) deblocking the diethylacetal and tetrahydrofuranyl groups by hydrolysis of the product of step (7) in .1 M aqueous hydrochloric acid-acetonitrile (2.5:1 v/v) for 30 minutes at 25°C to obtain 4'-deoxydesmycosin.

The 4'-deoxy derivatives of this invention, i.e. the compounds of formula (I) wherein $R^3$ is hydrogen, are readily prepared by procedures analogous to those described *supra*, using 4'-deoxy-OMT, 4'-deoxy-DOMT or 4'-deoxy-DH—DO—OMT as the starting material. These starting materials can be prepared via procedures outlined in *J. Antibiotics 34*, 1381—1384 (1981). Alternatively, deoxygenation at 4' may be accomplished in OMT, DOMT or DH—DO—OMT subsequent to modification of the C—20 and/or C—23 positions.

The formula (I) compounds which are ester derivatives are prepared by esterifying the respective C—20 and/or C—23-modified derivative on the 2',4', and/or 23-hydroxyl groups (when present) by treatment with acylating agents, using standard methods exemplified in the art. The preparation of 2'-O-ester derivatives of the C—20- and/or C—23-modified derivatives is accomplished by procedures similar to those described by Baltz *et al* in U.S. Patents 4,321,361 and 4,321,362. Esterification of the 2',4' and/or 23-hydroxyl groups of these modified derivatives may be accomplished by acylation of the hydroxyl groups using similar procedures as outlined in EP—A—0 082 002, EP—A—0 082 003 and US—A—4,396,613.

Alternatively, the formula (I) compounds which are esters may be prepared by starting with the appropriate esters of compounds *2—11*, prepared as described *supra*. Furthermore, it should be noted that the formula (I) ester compounds can be hydrolyzed to yield the corresponding formula (I) compounds, thus utilizing the esters as protecting groups during reactions to modify the C—20 and/or C—23 positions.

The C—20-modified derivatives of this invention form salts, particularly acid addition salts. These acid addition salts are also useful as antibiotics and are a part of this invention. In another aspect, such salts are useful as intermediates, for example, for separating and purifying the derivatives. In addition, the salts have an improved solubility in water.

Representative suitable salts include those salts formed by standard reactions with both organic and inorganic acids such as, for example, sulfuric, hydrochloric, phosphoric, acetic, succinic, citric, lactic, maleic, fumaric, palmitic, cholic, pamoic, mucic, D-glutamic, *d*-camphoric, glutaric, glycolic, phthalic, tartaric, formic, lauric, stearic, salicylic, methanesulfonic; benzenesulfonic, sorbic, picric, benzoic or cinnamic.

Pharmaceutically acceptable acid addition salts are an especially preferred group of salts of this invention.

Illustrative formula (I) compounds of this invention are listed in Table I.

TABLE I

Illustrative Formula (I) Compounds

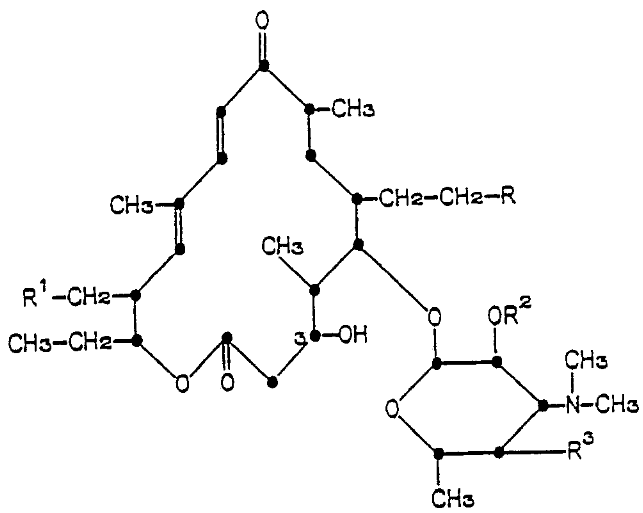

(I)

| Compound No. | R | $R^1$ | $R^2$ | $R^3$ |
|---|---|---|---|---|
| 26 | phenoxy | octahydroazocin-1-yl | H | —OH |
| 27 | (N-phenylacetyl)amino | " | " | " |
| 28 | pheylthio | azabicyclononanyl | " | " |
| 29 | chloro | 4-phenylpiperidino | " | " |
| 31 | phenoxy | hexahydroazepin-1-yl | " | mycarosyloxy |
| 35 | H | octahydroazocin1-yl | " | " |
| 37 | H | 4-hydroxypiperidino | " | —OH |
| 41 | H | 3-azabicyclononan-3-yl | " | " |
| 42 | H | 4-phenylpiperidino- | " | " |
| 45 | —OH | octahydroazocin-1-yl | " | H |
| 48 | H | octahydroazocin-1-yl | acetyl | acetoxy |
| 49 | —OH | octahydroazocin-1-yl | H | —OH |
| 50 | pyrrolidin-1-yl | OH | " | " |
| 51 | piperidin-1-yl | " | " | " |
| 52 | 4-hydroxypiperidin-1-yl | " | " | " |
| 53 | 4-phenylpiperidin-1-yl | " | " | " |
| 54 | hexahydroazepin-1-yl | " | " | " |
| 55 | octahydroazocin-1-yl | " | " | " |
| 56 | octahydro-1H-azonin-1-yl | " | " | " |
| 57 | decahydroazecin-1-yl | " | " | " |

TABLE I (continued)

Illustrative Formula (I) Compounds

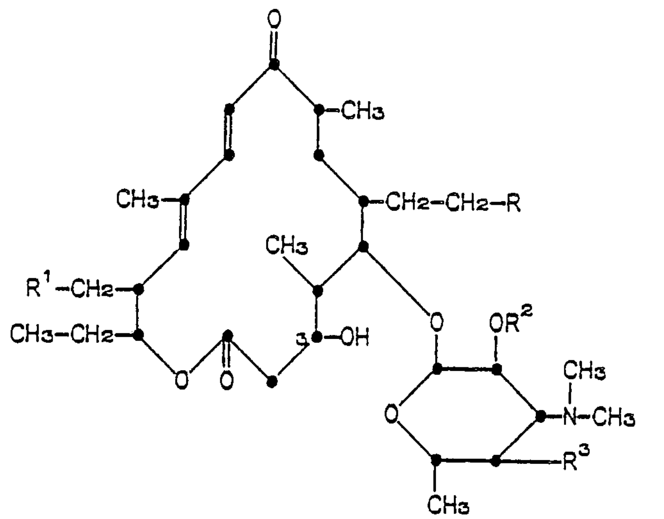

(I)

| Compound No. | R | $R^1$ | $R^2$ | $R^3$ |
|---|---|---|---|---|
| 58 | azacycloundecan-1-yl | " | " | " |
| 59 | axacyclotridecan-1-yl | " | " | " |
| 60 | 1,2,3,4-tetrahydro-quinolin-1-yl | " | " | " |
| 61 | 1,2,3,4-tetrahydroiso-quinolin-2-yl | " | " | " |
| 62 | 3-azabicyclononan-3-yl | " | " | " |
| 64 | octahydroazocin-1-yl | octahydroazocin-1-yl | " | " |
| 65 | 3-azabicyclononan-3-yl | octahydroazocin-1-yl | " | " |
| 66 | 3-azabicyclononan-3-yl | O-phenyl | " | " |
| 67 | morpholino | S-phenyl | " | " |
| 68 | octahydroazocin-1-yl | azido | " | " |
| 69 | octahydroazocin-1-yl | 3-azabicyclononan-3-yl | " | " |
| 70 | octahydroazocin-1-yl | OH | " | H |
| 71 | 3-azabicyclononan-3-yl | " | " | " |
| 72 | hexahydroazepin-1-yl | H | " | OH |
| 74 | 3-azabicyclononan-3-yl | OH | acetyl | acetoxy |

The derivatives of this invention inhibit the growth of pathogenic bacteria, especially gram-positive bacteria, and *Mycoplasma* species. Certain of the derviatives are active against some gram-negative bacteria, such as *Pasteurella* species. The minimal inhibitory concentrations (MIC's) at which illustrative compounds inhibit certain bacteria are given in Tables II and III. The MIC's in Table II were determined by standard agar-dilution assays. The MIC's in Table III were obtained using a conventional broth-dilution microtiter test.

TABLE II

Antibiotic Activity of Formula (I) Compounds[a]

| Test organism | Test Compound[b] 25 | 26 | 35 | 37 | 45 | 53 | 62 | 64 |
|---|---|---|---|---|---|---|---|---|
| *Staphylococcus aureus* X1.1 | 1 | 0.25 | 0.5 | 8 | 0.5 | 2 | 1 | 0.5 |
| *Staphylococcus aureus* V41[c] | 1 | 0.25 | 0.5 | 16 | 0.5 | 2 | 1 | 0.5 |
| *Staphylococcus aureus* X400[d] | 2 | 0.5 | 1 | 32 | 1 | 2 | 1 | 0.5 |
| *Staphylococcus aureus* S13E | 1 | 0.25 | 0.5 | 8 | 0.5 | 2 | 1 | 0.5 |
| *Staphylococcus epidermidis* EPI1 | 1 | 0.25 | 0.5 | 8 | 0.5 | 1 | 1 | 0.5 |
| *Staphylococcus epidermidis* EPI2 | 1 | 0.12 | 0.25 | 2 | 0.25 | 0.5 | 0.25 | 0.25 |
| *Streptococcus pyogenes* C203 | 2 | 0.12 | 0.12 | 32 | 0.25 | 2 | 2 | 0.125 |
| *Streptococcus pneumoniae* Park | 0.5 | 0.12 | 1 | 2 | 0.25 | 0.25 | 0.25 | 0.125 |
| *Streptococcus* Group D X66 | 1 | 2 | 1 | 64 | 2 | 2 | 4 | 0.5 |
| *Streptococcus* Group 9960 | 2 | 2 | 2 | 128 | 4 | 4 | 8 | 1 |
| *Haemophilis influenzae* C.L.[e] | 128 | 4 | 4 | 32 | 2 | 8 | 4 | 0.5 |
| *Haemophilus influenzae* 76[f] | — | 8 | 8 | 64 | 4 | 8 | 2 | 1 |
| *Escherichia coli* EC14 | | — | — | — | 64 | 32 | 32 | 4 |
| *Escherichia coli* TEM | | 8 | 16 | — | 8 | 8 | 8 | 2 |
| Klebsiella pneumoniae X26 | | 8 | 4 | 16 | 2 | 2 | 2 | 2 |
| Klebsiella pneumoniae KAE | | — | — | — | 64 | 64 | 64 | 8 |

[a]MIC in μg/ml
[b]Compound numbers from Table I
[c]Penicillin-resistant strain
[d]Methicillin-resistant strain
[e]Ampicillin-sensitive strain
[f]Ampicillin-resistant strain
[g]Compound not active at 128 μg/ml
[h]NT = not tested

TABLE III

Antibiotic Activity of Formula (I) Compounds[a]

| Test Organism | 26 | 35 | 37 | 48 | 49 |
|---|---|---|---|---|---|
| *Staphylococcus aureus* | 0.39 | 0.39 | 12.5 | 0.39 | 0.78 |
| *Streptococcus sp.* 80 | 0.78 | 0.78 | 1.56 | 0.195 | 1.56 |
| *Pasteurella multocida* 17E[c] | 3.12 | 0.78 | 25 | 1.56 | 3.12 |
| *Pasteurella multocida* 60A[d] | 6.25 | 0.78 | 12.5 | 1.56 | 0.78 |
| *Pasteurella multocida* 22A | 6.25 | 1.56 | 12.5 | 1.56 | 1.56 |
| *Pasteurella multocida* 40G | 3.12 | 1.56 | 12.5 | 1.56 | 0.39 |
| *Pasteurella multocida* 68C | 3.12 | 1.56 | 12.5 | 0.78 | 1.56 |
| *Pasteurella hemolytica* 22C | 1.56 | 0.78 | 25 | 0.78 | 0.78 |
| *Pasteurella hemolytica* 41D | 3.12 | 0.78 | 25 | 0.78 | 0.39 |
| *Pasteurella hemolytica* 23C | 3.12 | 1.56 | 25 | 1.56 | 0.78 |
| *Mycoplasma gallisepticum* | 0.097 | 0.78 | 6.25 | 0.78 | 0.39 |
| *Mycoplasma gallisepticum* 34159[e] | 50 | — | — | 50 | — |
| *Mycoplasma gallisepticum*41313[e] | 25 | — | 50 | 50 | 50 |
| *Mycoplasma synoviae* | 12.5 | — | 12.5 | 12.5 | 6.25 |
| *Mycoplasma hyorhinis* | 50 | 50 | — | — | 25 |

[a]MIC in μg/ml
[b]Compound numbers from Table I
[c]Bovine isolation
[d]Avian isolate
[e]Resistant strain
[f]MIC greater than 50 μg/ml

Some of the derivatives of this invention have shown *in vivo* antimicrobial activity against experimentally-induced infections in laboratory animals. When two doses of test compound were administered to mice experimentally infected with *S. pyogenes* C203, the activity observed was measured as an $ED_{50}$ value [effective dose in mg/kg to protect 50% of the test animals: see Warren Wick, et al., *J. Bacteriol. 81*, 233—235 (1961)]. $ED_{50}$ values observed for illustrative compunds are given in Table IV.

TABLE IV

$ED_{50}$ Values of Illustrative Formula (I) Compounds[a]

| | *Streptococcus pyrogenes* C203 | |
|---|---|---|
| Test Compound[b] | Subcutaneous | Oral |
| 35 | >12.5 | >100 |
| 37 | >10 | >100 |

[a]mg/mg × 2; doses given 1 and 4 hours post-infection
[b]Compound numbers from Table I.

Certain of the formula (I) compounds of this invention have also shown *in vivo* activity against infections induced by gram-negative bacteria. Table V summarizes the results of tests in which illustrative

compounds were evaluated against a *Pasteurella* infection in one-day-old chicks. The compounds were administered parenterally after challenge of the chicks with *Pasteurella multocida* (0.1 ml of a $10^{-4}$ dilution of a twenty-hour tryptose broth culture of an avian *P. multocida* given subcutaneously). In these tests, unless indicated otherwise, all non-medicated infected chicks died within 24 hours of *Pasteurella* challenge. In the tests summarized in Table V, the compounds were administered by subcutaneous injection at a dosage of 30 mg/kg, 1 and 4 hours post-challenge of the chicks with *P. multocida.*

TABLE V

Activity of Formula (I) Compounds Administered Subcutaneously to *Pasteurella multocida*-Infected Chicks[a]

| Test Compound[b] | Number of Deaths/Number Treated |
|---|---|
| 26 | 10/10 |
| 35 | 8/10 |
| 37 | 10/10 |

[a]Administered subcutaneously; 30 mg/kg × 2
[b]Compound numbers from Table I

The compounds which are prefered for *in vivo* activity against gram-positive microorganisms are those formula (I) compounds wherein R is —N($R^6$)$_2$. Another preferred group are the formula (I) compounds wherein $R^1$ is —OH or —OAr and Ar is a group (iii) substituent. Still another group of compounds preferred for *in vitro* activity against gram-positive bacteria and for activity against *Mycoplasma* species are the formula (I) compounds wherein $R^1$ is $R^9$.

This invention also relates to methods of controlling infections caused by gram-positive bacteria and *Mycoplasma* species. In carrying out the methods of this invention, an effective amount of a specified formula (I) compound is administered parenterally to an infected or susceptible warm-blooded animal.

The dose which is effective to control the infection will vary with the severity of the infection and the age, weight, and condition of the animal. The total dose required for protection parenterally will generally, however, be in the range of from 1 to 100 mg/kg and preferably will be in the range of from 1 to 50 mg/kg. Suitable dosage regimens can be constructed.

In another aspect, this invention relates to compositions useful for the control of infections caused by gram-positive bacteria and *Mycoplasma* species. These compositions comprise a specified compound of formula (1) together with a suitable vehicle. Compositions may be formulated for parenteral administration by methods recognized in the pharmaceutical art.

Effective injectable compositions containing these compounds may be in either suspension or solution form. In the preparation of suitable formulations it will be recognized that, in general, the water solubility of the acid addition salts is greater than that of the free bases. Similarly, the bases are more soluble in dilute acids or in acidic solutions than in neutral or basic solutions.

In the solution form the compound is dissolved in a physiologically acceptable vehicle. Such vehicles comprise a suitable solvent, preservatives such as benzyl alcohol, if needed, and buffers. Useful solvents include, for example, water and aqueous alcohols, glycols, and carbonate esters such as diethyl carbonate. Such aqueous solutions contain, in general, no more than 50% of the organic solvent by volume.

Injectable suspension compositions require a liquid suspending medium, with or without adjuvants, as a vehicle. The suspending medium can be, for example, aqueous polyvinylpyrrolidone, inert oils such as vegetable oils or highly refined mineral oils, or aqueous carboxymethylcellulose.

Suitable physiologically acceptable adjuvants are necessary to keep the compound suspended in suspension compositions. The adjuvants may be chosen from among thickeners such as carboxymethylcellulose, polyvinylpyrrolidone, gelatin, and the alginates. Many surfactants are also useful as suspending agents. Lecithin, alkylphenol polyethylene oxide adducts, naphthalenesulfonates, alkylbenzenesulfonates, and the polyoxyethylene sorbitan esters are useful suspending agents.

Many substances which affect the hydrophilicity, density, an surface tension of the liquid suspending medium can assist in making injectable suspensions in individual cases. For example, silicone antifoams, sorbitol, and sugars can be useful suspending agents.

In order to illustrate more fully the operation of this invention, the following examples are provided:

Preparation 1

2′,4′-Di-O-acetyl-20-dihydro-OMT

20-Dihydro-OMT (3.1 g, 5.2 mmol) was dissolved in acetone (100 ml) and was treated over a five-minute period with acetic anhydride (2.0 ml, 21.2 mmol). After stirring for nine hours at room temperature,

the reaction mixture was quenched into saturated sodium bicarbonate solution (500 ml) and the product was extracted into dichloromethane (2 × 250 ml). The combined dichloromethane extracts were dried (sodium sulfate) and filtered and the filtrate was evaporated under reduced pressure. The residue was dried *in vacuo* overnight to yield 3.4 g (96%) of 2',4'-di-O-acetyl-20-dihydro-OMT.

Preparation 2

23-Iodo-20,23-dideoxy-20-dihydro-OMT

20-Deoxy-20-dihydro-OMT (2.0 g, 3.4 mmol), tetrabutylammonium iodide (3.8 g, 10.3 mmol) and s-collidine (1.36 ml, 10.3 mmol) were dissolved in dichloromethane (40 ml). The solution was cooled to −78° under an argon atmosphere and then was treated dropwise with triflic anhydride (0.7 ml). After 5 minutes at −78°, the cooling bath was removed and the solution was stirred for 30 minutes at room temperature. Since tlc[a] analysis showed unreacted starting material was still present, the solution was cooled to −78° again and then treated with additional triflic anhydride (0.03 ml). The cooling bath was again removed and the reaction was stirred at room temperature for 30 minutes. The solution was extracted with saturated sodium bicarbonate solution, dried ($Na_2SO_4$) and filtered. The filtrate was evaporated to dryness and the crude product was purified by flash chromatography on silica gel, eluting with a linear gradient of dichloromethane (1 L) and 5% methanol in dichloromethane (1 L). Fractions containing the desired product were located by tlc analysis, combined and evaporated under reduced pressure to yield 2.0 g of 23-iodo-20,23-dideoxy-20-dihydro-OMT.

Preparation Example 1

2',4'-Di-O-acetyl-20-O-phenylacetyl-20-dihydro-OMT and 2',4'-di-O-acetyl-20,23-di-O-phenylacetyl-20-dihydro-OMT

2',4'-Di-O-acetyl-20-dihydro-OMT (3.0 g, 4.4 mmol) was dissolved in dichloromethane (50 ml) and pyridine (2 ml). The solution was cooled to −78° and treated dropwise with phenylacetyl chloride (0.725 ml, 5.5 mmol) over a 2-minute period with vigorous stirring. AFter 15 minutes at −78°, the cooling bath was removed and the solution was stirred at room temperature for six hours. The solution was then poured into saturated sodium bicarbonate solution (100 ml) and the product was extracted into dichloromethane (2 × 50 ml). The combined dichloromethane extracts were dried (sodium sulfate) and filtered and the filtrate was evaporated. The residue (4 g) was separated on a Waters Prep 500 chromatograph, eluting with a linear gradient of toluene (4 l) and ethyl acetate (4 l). Fractions containing the desired products wre located by tlc analysis, combined and evaporated under reduced pressure to yield 2.3 g of 2',4'-di-O-acetyl-20-O-phenylacetyl-20-dihydro-OMT and 0.6 g of 2',4'-di-O-acetyl-20,23-di-O-phenylacetyl-20-dihydro-OMT.

Preparation Example 2

20-Dihydro-23-O-phenylpropionyl-OMT

23-O-Phenylpropionyl-OMT (1.9 g, 2.6 mmol) was dissolved in 1:1 isopropanol:water (30 ml). Sodium borohydride (0.025 g, 0.65 mmol) was added to this solution and the reaction was stirred for 0.5 h. The pH of the reaction was adjusted from pH 10.5 to pH 7.0 with 1N sulfuric acid. The solution was concentrated to aqueous under reduced pressure and saturated $NaHCO_3$ solution was added. The product was extracted into dichloromethane and the extracts were dried ($Na_2SO_4$) and filtered. The filtrate was evaporated under reduced pressure to yield 1.75 g (92%) of the title compound as a white foam.

Example 3

20-Dihydro-23-Octahydroazocin-1-yl-23-deoxy-OMT

23-Octahydroazocin-1-yl-OMT (900 mg, 1.3 mmol) was reduced with sodium borohydride (12 mg, 0.33 mmol) in 1:1 isopropanol-water (15 ml) as described in example 17, yielding 815 mg (90%) of the 20-dihydro derivative.

Preparation Example 4

20-O-Phenyl-20-dihydro-23-O-phenylpropionyl-OMT

20-Dihydro-23-O-phenylpropionyl-OMT (1.7 g, 2.3 mmol), triphenylphosphine (1.2 g, 4.6 mmol) and phenol (0.43 g, 4.6 mmol) were dissolved in tetrahydrofuran (45 ml) under a nitrogen atmosphere. The solution was cooled in an ice bath and then was treated dropwise with diethyl azodicarboxylate (0.8 g, 4.6 mmol). After 5 minutes, the cooling bath was removed and the solution was stirred for 2 h at room temperature. Since tlc analysis of the reaction indicated the presence of unreacted starting material, one-half of the initial amounts (2.3 mmol) of triphenylphosphine, phenol and diethyl azodicarboxylate were each added. After stirring for another 30 minutes, methanol (10 ml) was added to decompose excess reagent and the solution was evaporated under reduced pressure. The residual oil was treated with toluene and the insoluble material was filtered. The filtrate was evaporated under reduced pressure and the residue was separated by flash chromatography on silica gel, eluting step-wise with mixtures of methanol-dichloromethane as follows: 400 ml of 0%, 250 ml of 2%, 250 ml of 4%, 750 ml of 6% and 250 ml of 8%

---

[a]thin layer chromatography

methanol in dichloromethane. Fractions containing the desired product were located by tlc analysis, combined and evaporated under reduced pressure to yield 0.26 g of 20-O-phenyl-20-dihydro-23-O-phenylpropionyl-OMT.

Example 5

20-O-Phenyl-20-dihydro-23-Octahydroazocin-1-yl-OMT

20-Dihydro-23-octahydroazocin-1-yl-OMT (800 mg, 1.2 mmol), triphenylphosphine (940 mg, 3.6 mmol) and phenol (340 mg, 3.6 mmol) were dissolved in tetrahydrofuran (20 ml). The solution was treated with diethyl azodicarboxylate (630 mg, 3.6 mmol), stirred for 1 hour, and worked up as described in example 19. The crude product was purified by flash chromatography on silica gel, eluting stepwise with mixtures of methanol-dichloromethane as follows: 400 ml of 0%, 250 ml of 2%, 500 ml of 3%, 250 ml each of 4%, 6%, 8%, 12% and 16% methanol in dichloromethane. Fractions containing the desired product were located by tlc analysis, combined and evaporated to yield 90 mg of the title compound.

Example 6

23-Octahydroazocin-1-yl-20,23-dideoxy-20-dihydro-OMT

23-Iodo-20,23-dideoxy-20-dihydro-OMT (69 mg) and heptamethyleneimine (0.05 ml) were dissolved in acetonitrile (2 ml) and the solution was refluxed for 2 h under an argon atmosphere. The solution was cooled to room temperature and poured into saturated sodium bicarbonate solution (10 ml). The product was extracted into dichloromethane and the extracts were dried ($Na_2SO_4$) and filtered. The filtrate was evaporated and the residue was separated by preparative tlc on a 20 × 20 cm, 2 mm thick plate of silica gel (E. Merck), developing with dichloromethane-methanol-conc. ammonium hydroxide (90:10:2). The band on the silica gel plate was located by UV light and was scraped from the plate, dried *in vacuo* to remove solvent and then eluted with dichloromethane-methanol (1:1, 50 ml) for 45 minutes. The mixture was filtered and the filtrate was evaporated to dryness to yield 65 mg of the title compound.

Example 7

23-(4-Hydroxypiperidino)-20,23-dideoxy-20-dihydro-OMT

23-Iodo-20,23-dideoxy-20-dihydro-OMT (1.1 g, 1.6 mmol) and 4-hydroxypiperidine (0.32 g, 3.2 mmol) were dissolved in acetonitrile (20 ml) and refluxed under an argon atmosphere for 2 h. Additional 4-hydroxypiperidine (300 mg) was added to consume unreacted starting material and the solution was refluxed for an additional 3 h. The solution was cooled to room temperature and then evaporated under reduced pressure. The residue was dissolved in dichloromethane, extracted with saturated sodium bicarbonate solution, dried ($Na_2SO_4$) and filtered. The filtrate was evaporated and the residue was purified by flash chromatography on silica gel, eluting with a linear gradient of dichloromethane (1 l) and 12% methanol in dichloromethane (1 l). Fractions containing the desired product were located by tlc analysis, combined and evaporated to yield 865 mg of the title compound.

Preparation Example 8

23-O-(2,3-Dimethoxyphenyl)-20-deoxy-20-dihydro-OMT

20-Deoxy-20-dihydro-OMT (3.0 g, 5.15 mmol), triphenylphosphine (2.7 g, 10.3 mmol) and 2,3-dimethoxyphenol (1.59 g, 10.3 mmol) were dissolved in tetrahydrofuran (150 ml) under an argon atmosphere. The solution was treated with diethyl azodicarboxylate (1.7 ml, 10.3 mmol) and then was stirred for 40 minutes at room temperature. Methanol (2 ml) was added to deompose excess reagent and the solution was evaporated to dryness under reduced pressure. The residue was taken up in toluene and the insoluble material was filtered. The filtrate was extracted with saturated sodium bicarbonate solution, dried ($Na_2SO_4$) and filtered and the filtrate was evaporated to dryness. The residue was purified by chromatography on silica gel (Waters Prep 500), eluting with dichloromethane (2 l) followed by a linear gradient of dichloromethane (2 l) and 10% methanol in dichloromethane (2 l); the column was finally eluted with 2 l of the latter solvent. Fractions containing the desired product were located by tlc analysis, combined and evaporated to yield 2.06 g (54%) of the title compound.

Example 9

23-O-(3-Pyridyl)-20,23-dideoxy-20-dihydro-OMT

20-Deoxy-20-dihydro-OMT (3.0 g, 5.15 mmol), triphenylphosphine (2.7 g, 10.3 mmol) and 3-hydroxypyridine (979 mg, 10.3 mmol) were dissolved in tetrahydrofuran (50 ml) under an argon atmosphere and treated with diethyl azodicarboxylate (1.7 ml, 10.3 mmol). After workup and chromatography as described in example 8, 0.63 g of the title compound was obtained.

Example 10

20-DH-DO-20-[3-Azabicyclo(3.2.2)-nonan-3-yl]-OMT

OMT (3.0 g, 50 mmoles) was dissolved in anhydrous methanol (15 ml). 3-Azabicyclo[3.2.2]-nonane (1.25 g, 10 mmoles) was dissolved in anhydrous methanol (15 ml) and filtered to remove a white impurity. The filtrate was added to the OMT solution and the resulting solution was stirred for 5—10 minutes at room temperature in the presence of molecular sieves (3A). $NaBH_3CN$ (0.63 g, 10 mmoles) was added, and the

reaction was stirred at room temperature for 17 hours. The reaction mixture was filtered, and the filtrate was evaporated under vacuum to give a foam which was redissolved in ethyl acetate (150 ml). The ethyl acetate solution was washed with water (150 ml) and separated. A major portion of the product was then extracted from the ethyl acetate solution with 0.5*M* $NaH_2PO_4$ buffer (150 ml, pH 6.5). The phosphate buffer solution was evaporated under vacuum to remove residual ethyl acetate. The pH of the buffer solution was adjusted to about 11 with 5N NaOH, forming a white precipitate which was collected by filtration and dried to give 2.0 g (58% yield) of 20-DH-DO-20-[3-azabicyclo(3.2.2)nonan-3-yl]OMT. [Titration $pK_a$ values: 7.7 and 9.3; FDMS parent ion ($M^+$ + 1) = 707].

## Example 11

20-DH-DO-20-(4-Phenylpiperidin-1-yl)-OMT

OMT (5.97 g, 10 mmoles), 4-phenylpiperidine (3.22 g, 20 mmoles), $NaBH_3CN$ (1.25 g, 20 mmoles) and methanol (60 ml) were reacted using the procedure of Example 1, but substituting a pH 4.5 buffer for extraction, to give 3.7 g of the title compound [FDMS parent ion ($M^+$ + 1) = 743].

## Example 12

20-DH-DO-23-Deoxy-20,23-di(octahydroazocin-1-yl)-OMT

20,23-di-iodo-OMT (1.2 g., 1.5 mmoles) was dissolved in acetonitrile (20 ml.). Heptamethyleneimine (1.7 g., 19 ml., 15 mmoles) was added to this solution, and the reaction mixture was stirred at reflux for 1.5 hours. Volatiles were removed, and the resulting red oil was dissolved in $CH_2Cl_2$ (150 ml.). This solution was washed with saturated $NaHCO_3$ solution (100 ml.) and the $CH_2Cl_2$ phase was separated and dried over $Na_2SO_4$, filtered, and evaporated under vacuum. The residue obtained was subjected to flash column chromatography on silica gel 60 packed in $MeOH/CH_2Cl_2$ (1:9). The column was eluted stepwise with $MeOH/CH_2Cl_2$ as follows: 300 ml. of 1:9, 500 ml. of 1:4, 250 ml. of 3:7, 250 ml. of 2:3, 500 ml. of 1:1, and 500 ml. of 7:3. The desired fractions were combined to give 221 mg. (19% yield) of 20-DH-DO-23-deoxy-20,23-di(octahydroazocin-1-yl)-OMT as a white foam. [Titration $pK_a$ values: 6.9, 8.05, 8.9; FDMS parent ion ($M^+$ + 1) = 790].

## Examples 13—35

The following compounds can be prepared by the methods of the preceding examples.

20-DH-DO-20-(octahydroazocin-1-yl)DMT
20-DH-DO-20-(piperidin-1-yl)DMOT
20-DH-DO-20-(piperidin-1-yl)DOMT
20-DH-DO-20-(4-hydroxypiperidin-1-yl)DOMT
20-DH-DO-20-(decahydroazecin-1-yl)OMT
20-DH-DO-20-(octahydroazocin-1-yl)DOMT
20-DH-DO-20-(azacyclotridecan-1-yl)OMT
20-DH-DO-20-(hexahydroazepin-1-yl)DMT
20-DH-DO-20-(1,2,3,4-tetrahydroisoquinolin-2-yl)OMT
20-DH-DO-20-(1,2,3,4-tetrahydroquinolin-1-yl)OMT
20-DH-DO-20-(azacycloundecan-1-yl)OMT
20-DH-DO-20-(4-methylpiperidin-1-yl)OMT
20-DH-DO-20-(pyrrolidin-1-yl)DMT
20-DH-DO-20-(octahydro-1H-azonin-1-yl)OMT
20-DH-DO-20-(octahydroazocin-1-yl)DMOT
20-DH-DO-20-(octahydroazocin-1-yl)DOMT
20-DH-DO-20-(4-phenylpiperidin-1-yl)DMT
20-DH-DO-20-(4-phenylpiperidin-1-yl)-4′-deoxy-OMT
20-DH-DO-20-(decahydroazecin-1-yl)-4′-deoxy-OMT
20-DH-DO-20-(hexahydroazepin-1-yl)-4′-deoxy-OMT
20-DH-DO-20-(1,2,3,4-tetrahydroisoquinolin-2-yl)DOMT
20-DH-DO-20-(decahydrocyclopent[c]azepin-1-yl)OMT
20-DH-DO-20-(7-azabicyclo[2.2.1]heptan-1-yl)OMT

## Example 36

Injectable Formulations

A) A formula (I) base is added to propylene glycol. Water and benzyl alcohol are added so that the solution contains 50% (by volume) propylene glycol, 4% (by volume) benzyl alcohol, and 200 mg/ml of a formula (I) base.

B) A solution is prepared as described in Section A except that the solution contains 50 mg/ml of a formula (I) base.

C) A solution is prepared as described in Section A except that the solution contains 350 mg/ml of a formula (I) base.

D) A solution is prepared as described in Section A except that the solution contains 500 mg/ml of a formula (I) tartrate.

E) A suspension is prepared by adding a finely ground formula (I) compound to carboxymethyl cellulose with thorough mixing so that the suspension contains 200 mg of the formula (I) base per ml of suspension.

Often the most practical way to administer the compounds is by formulation into the feed supply or drinking water. A variety of feeds, including the common dry feeds, liquid feeds, and pelleted feeds, may be used.

The methods of formulating drugs into animal feeds are well-known. A preferred method is to make a concentrated-drug premis which in turn is used to prepare medicated feeds. Typical premixes may contain from 1 to 200 grams of drug per 0.453 kg (pound) of premix. Premixes may be either liquid or solid preparations.

The final formulation of feeds for animals or poultry will depend upon the amount of drug to be administered. The common methods of formulating, mixing, and pelleting feeds may be used to prepare feeds containing a compound of formula (I).

Many substances which affect the hydrophilicity, density, and surface tension of the liquid suspending medium can assist in making injectable suspensions in individual cases. For example, silicone antifoams, sorbitol, and sugars can be useful suspending agents.

SUPPLEMENTAL DATA FOR COMPOUNDS
PREPARED IN THE EXAMPLES

| Example | Field Desorption Mass Spectrum (parent ion) | UV $\lambda_{max}(\varepsilon)$ (ETOH) | IR Most Intense $\gamma$ co ($CHCl_3$) | NMR Selected $\delta$ ($CDCl_3$) |
|---|---|---|---|---|
| 18 | 695 | — | — | 1.46 broad singlet, $CH_2$ protons-non amine adjacent-form heptamethylene amino group. |
| 20 | 771 | 279 (11,000)<br>220 (11,250) | — | 6.88—6.98 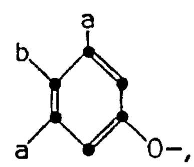 m |
| 21 | 709 | 279 nm (21,400) | 1743 $cm^{-1}$ | 7.22—7.32 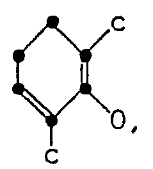 m |
| 24 | 679 | 284 (19,000) | 1715 $cm^{-1}$ | 1.48,1.52($CH_2$) |
| 25 | 666 | 283 (17,500) | 1715 $cm^{-1}$ | no data |
| 27 | 661 | 278 (24,000)<br>216 (9,700) | 1716 $cm^{-1}$ | 8.08—8.39 (aromatic) |

**Claims for the Contracting States: BE CH DE FR GB IT LI LU NL SE**

1. A macrolide of the formula (I):

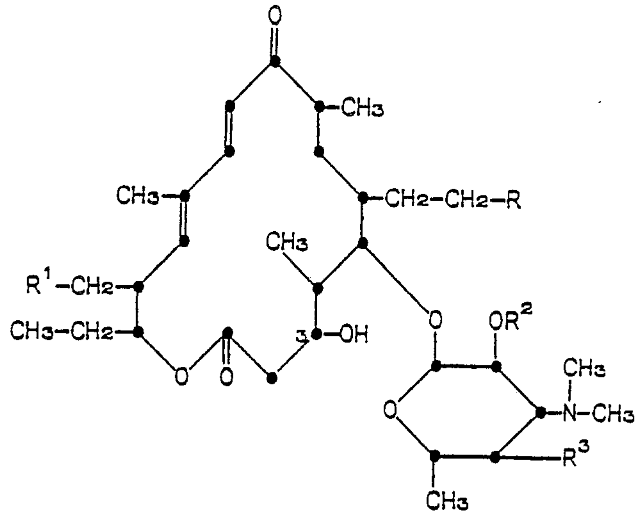

(I)

wherein

R is hydrogen, iodo, bromo, chloro, fluoro, cyano, —$OR^4$, —OAr, —$SR^5$, azido, —$NR^6R^7$, N-phthalimido or $R^9$;

$R^1$ is i) hydrogen or —OH; ii) chloro, fluoro, bromo, iodo —OAr, —O-tetrahydrofuranyl, —O-tetrahydropyranyl, —$SR^5$, azido, —$NR^6R^7$, or N-phthalimido; or $R^9$;

$R^9$ is i) a monocyclic amino group of the formula —$N(CH_2)_n$ which is optionally substituted at one or more of the carbon atoms by a $C_1$—$C_3$-alkyl, hydroxyl, methoxyl, ethoxyl, —$N(R^8)_2$,

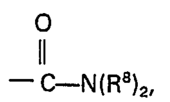

carbomethoxy, carboethoxy, or phenyl group; and n is an integer from 4 through 15; ii) a monocyclic saturated or unsaturated nitrogen-containing heterocyclic ring bonded through the nitrogen atom, said ring having 1) from 5 to 7 ring atoms which include up to 3 additional heteroatoms selected from nitrogen, oxygen and sulfur, and 2) up to 3 substituent groups selected from methyl, ethyl and phenyl; or iii) a bicyclic or tricyclic secondary amino group selected from 1,2,3,4-tetrahydroquinolin-1-yl; decahydroquinolin-1-yl; 1,2,3,4-tetrahydroisoquinolin-2-yl; decahydroisoquinolin-2-yl; indolin-1-yl; isoindolin-2-yl; decahydrocyclohepta[b]-pyrrol-1-yl; decahydrocyclohepta[c]-pyrrol-2-yl; decahydrocyclopent[c]-azepin-2-yl; decahydrocyclopent[d]azepin-3-yl; 2,3,4,5-tetrahydro-1H-2-benzazepin-2-yl; 2,3,4,5-tetrahydro-1H-3-benzazepin-3-yl; azabicycloheptanyl; azabicyclooctanyl; azabicyclononanyl; azabicyclodecanyl or azatricyclodecanyl;

$R^2$ is hydrogen, optionally substituted $C_1$—$C_5$-alkanoyl or optionally substituted benzoyl, phenylacetyl or phenylpropionyl;

$R^3$ is hydrogen, hydroxyl, optionally substituted $C_1$—$C_5$-alkanoyloxy or optionally substituted benzoyloxy, phenylacetoxy or phenylpropionyloxy or

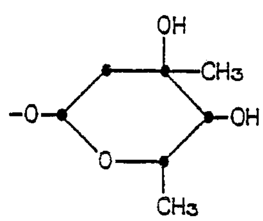

;

(mycarosyloxy)

$R^4$ is hydrogen, optionally substituted $C_1$—$C_4$-alkyl, cyclohexyl, optionally substituted benzyl, phenethyl or phenoxyethyl;

Ar is i) phenyl, derivatized phenyl, or naphthyl; ii) an optionally substituted heteroaryl group selected

from pyridinyl, pyrimidinyl, pyridazinyl, pyrazinyl, triazinyl, indolyl, isoquinolinyl, quinolinyl, quinazolinyl, cinnolinyl, quinoxalinyl, phthalazinyl, benzotriazolyl, benzoxazolyl, benzimidazolyl, carbazolyl, or acridinyl; or

iii) optionally substituted $C_1$—$C_5$-alkanoyl; optionally substituted benzoyl, phenylacetyl, phenylpropionyl, phenoxyacetyl or phenylthioacetyl; methanesulfonyl; trifluoromethanesulfonyl; or optionally substituted phenylsulfonyl;

$R^5$ is optionally substituted $C_1$—$C_4$-alkyl; cyclohexyl; optionally substituted phenyl, benzyl or phenethyl; or an optionally substituted heteroaryl group selected from imidazolyl, pyrazolyl, pyridinyl, pyrimidinyl, pyrazinyl, pyridazinyl, triazinyl, triazolyl, tetrazolyl, oxazolyl, isoxazolyl, oxadiazolyl, thiazolyl, isothiazolyl, thiadiazolyl, thienyl and furanyl;

$R^6$ is hydrogen, optionally substituted $C_1$—$C_6$-alkyl, phenyl, benzyl, phenethyl or $C_3$—$C_8$-cycloalkyl;

$R^7$ is an $R^6$ group or optionally substituted $C_1$—$C_5$-alkanoyl, optionally substituted benzoyl, phenylacetyl, phenylpropionyl, phenoxyacetyl or phenylthioacetyl, or alkoxycarbonyl; and

$R^8$ is hydrogen, methyl, ethyl, n-propyl or isopropyl or the $R^8$ groups taken together form a polymethylene moiety such that —$N(R^8)_2$ constitutes a cyclic amino group selected from pyrrolidinyl, piperidinyl, hexahydroazepinyl or octahydroazocinyl;

provided 1) that, when $R^4$ is hydrogen, $R^1$ cannot be hydrogen or —OH; 2) that, when R or $R^1$ is —$NHR^6$ or $R^4$ or $R^8$ is hydrogen, $R^2$ must be hydrogen, $R^3$ must be hydrogen, hydroxyl, or mycarosyloxy and Ar cannot be a type (iii) substituent; 3) that, when $R^2$ is hydrogen, $R^3$ must be hydrogen, hydroxyl or mycarosyloxy; 4) that at least one of R and R' is $R^9$; 5) that when R, is H or OH, $R^9$ is not a monocyclic saturated or unsaturated nitrogen-containing heterocyclic ring as defined under ii), or a physiologically acceptable salt thereof.

2. A macrolide of formula (I) as claimed in claim 1 wherein R is $R^9$.

3. A macrolide of formula (I) as claimed in claim 1 wherein R is hydrogen, hydroxy, phenoxy, N-phthalimido, phenylacetoxy, 3-azabicyclo(3.2.2)-nonan-3-yl, morpholino, 4-phenylpiperidin-1-yl, or octahydroazocin-1-yl.

4. A macrolide of formula (I) as claimed in claim 1 wherein $R^1$ is hydrogen, hydroxy, phenoxy, acetoxy, phenylacetoxy, N-phthalimido, phenylpropionyloxy, phenylthio, octahydroazocin-1-yl, 4-hydroxypiperidino, 2,3-dimethoxyphenoxy, 3-pyridyloxy, or m-dimethylaminophenoxy.

5. A process for preparing a macrolide of formula (I), as defined in claim 1, or a physiologically acceptable salt thereof which comprises:

(a) reducing a starting macrolide of formula (II) wherein Q is formyl and $Q^1$ is $R^1$, provided $R^1$ is not hydroxyl, to give a macrolide of formula (I) wherein R is hydroxyl;

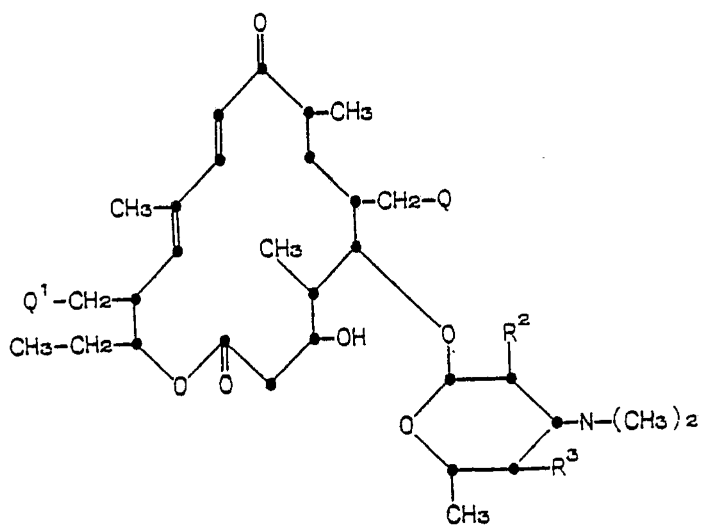

(II)

(b) reacting a starting macrolide of formula (II) wherein Q is formyl and $Q^1$ is $R^1$ with an amine of the formula $HNR^6R^6$ or $HR^9$ in the presence of a reducing agent to give a macrolide of formula (I) wherein R is $NR^6R^6$ or $R^9$, or

(c) reacting a starting macrolide of formula (II) wherein Q is —$CH_2OH$ and $Q^1$ is $R^1$ with diethylazodicarboxylate or dimethylazodicarboxylate, triphenylphosphine, and a reagent selected from

(i) an azide transfer agent, to give a macrolide of formula (I) wherein R is azido,

(ii) phthalimide to give a macrolide of formula (I) wherein R is phthalimido,

(iii) a phenol of formula ArOH to give a macrolide of formula (I) wherein R is —OAr, where Ar is a category 1) or ii) Ar group,

(iv) an alkyl halide or polyhalide to give a macrolide of formula (I) wherein R is Cl, Br, or I, or

(v) a mercaptan of formula $HSR^5$ to give a macrolide of formula (I) wherein R is $SR^5$, or

(vi) a carboxylic or sulfonic acid of the formula ArOH, where Ar is a category (iii) Ar group, to give a macrolide of formula (I) wherein R is OAr,

(d) reacting a starting macrolide of formula (II) wherein Q is $—CH_2OH$ and $Q^1$ is $R^1$ with triphenylphosphine and a halogen source to give a macrolide of formula (I) wherein R is Cl, Br, or I,

(e) reacting a starting material of formula (II) wherein Q is $—CH_2OH$ and $Q^1$ is $R^1$ with an acylating agent derived from a carboxylic or sulfonic acid of formula ArOH, where Ar is a category (iii) Ar group to give a macrolide of formula (I) wherein R is OAr, or

(f) reacting a starting macrolide of formula (II) wherein Q is $—CH_2L$ where L is a leaving group and $Q^1$ is $R^1$ or a leaving group with

(i) an alkali metal azide or halide or a tetraalkylammonium azide or fluoride where alkyl is methyl, ethyl, propyl or butyl, to give a macrolide of formula (I) wherein R is azido, F, Cl, Br, or I, or

(ii) a mercaptide ion of formula $R^5S—$ to give a macrolide of formula (I) wherein R is $R^5S—$, or

(iii) an amine of the formula $NR^6R^6$ or $HR^9$ to give a macrolide of formula (I) wherein R is $NR^6R^6$ or $R^9$, or

(iv) a source of cyanide ion to give a macrolide of formula (I) wherein R is —CN;

(v) an alcohol of the formula $HOR^4$ and a source of silver ion to provide a macrolide of formula (I) wherein R is $OR^4$ where $R^4$ is other than hydrogen

(g) hydrolyzing a macrolide of formula (II) wherein Q is $CH_2R$ and $Q^1$ is

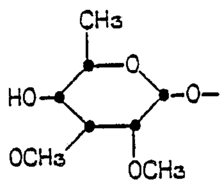 , 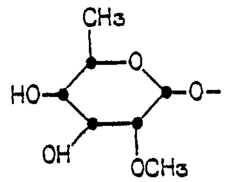 , or 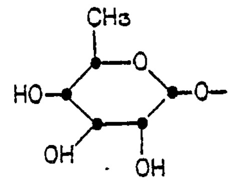

to give a macrolide of formula (I) wherein $R^1$ is hydroxyl, or

(h) removing the hydroxy protecting group from a macrolide of formula (II) wherein Q is $—CH_2R$ and $Q^1$ is protected hydroxy or

(i) reacting a starting macrolide of formula (II) wherein Q is $—CH_2R$ and $Q^1$ is hydroxyl with diethylazodicarboxylate or dimethylazodicarboxylate, triphenylphosphine, and a reagent selected from

(i) an azide transfer agent to give a macrolide of formula (I) wherein $R^1$ is azido,

(ii) phthalimide to give a macrolide of formula (I) wherein $R^1$ is phthalimido,

(iii) a phenol of formula ArOH where Ar is a category (i) or (ii) Ar group to give a macrolide of formula (I) wherein $R^1$ is —OAr.

(iv) an alkyl halide or polyhalide to give a macrolide of formula (I) where $R^1$ is Cl, Br, or I, or

(v) a mercaptan of formula $HSR^5$ to give a macrolide of formula (I) wherein R is $SR^5$, or

(vi) a carboxylic or sulfonic acid of the formula ArOH, where Ar is a category (i) Ar group, to give a macrolide of formula (I) wherein $R^1$ is OAr; or

(j) reacting a macrolide of formula (II) wherein Q is $—CH_2R$ and $Q^1$ is hydroxyl with an acylating agent derived from a carboxylic or sulfonic acid of formula ArOH where Ar is a category (iii) Ar group to give a macrolide of formula (I) wherein $R^1$ is OAr or

(k) reacting a macrolide of formula (II) wherein Q is R and $Q^1$ is hydroxyl with triphenylphosphine and a halogenating agent to give a macrolide of formula (I) wherein $R^1$ is Cl, Br or I, or

(l) reacting a macrolide of formula (II) wherein Q is $—CH_2R$ and $Q^1$ is a leaving group with

(i) an alkali metal azide or halide or a tetraalkylammonium azide or fluoride where alkyl is methyl, ethyl, propyl, or methyl to give a macrolide of formula (I) wherein $R^1$ is azido, F, Cl, Br, or I,

(ii) a mercaptide ion of formula $R^5S—$ to give a macrolide of formula I wherein $R^1$ is $R^5S—$, or

(iii) an amine of the formula $HNR^6R^6$ or $HR^9$ to give a macrolide of formula (I) wherein $R^1$ is $HR^6R^6$ or $R^9$, or

(m) reducing a macrolide of formula (II) wherein Q is $—CH_2N_3$ and $Q^1$ is $R^1$ or Q is R and $Q^1$ is azido to give a macrolide of formula (I) wherein R is $—CH_2NH_2$ or $R^1$ is amino, or

(n) acylating a macrolide of formula (II) wherein Q is $—CH_2NHR^6$ or $R^1$ is $NHR^6$ to give a macrolide of formula (I) wherein R is $—CH^2NHR^7$ or $R^1$ is $—NR^6R^7$, or

(o) esterifying a macrolide of formula (I), or

(p) salifying a macrolide of formula (I), or

(q) hydrolyzing a macrolide of formula (I) wherein $R^3$ is mycarosyloxy in acid solution at a pH below 4 to give a macrolide of formula (I) wherein $R^3$ is hydroxy, or

(r) deoxygenating a macrolide of formula (I) wherein $R^3$ is hydroxy to give a macrolide of formula (I) wherein $R^3$ is hydrogen.

(s) reacting a macrolide of formula (II) wherein Q is $CH_2I$ and $Q^1$ is $R^1$ with a reducing agent to give a compound of formula (I) wherein R is hydrogen, or

(t) reacting a macrolide of formula (II) wherein Q is $—CH_2—$ sulfonate and $Q^1$ is $R^1$ with a source of iodide ion to give a macrolide of formula (I) wherein R is iodo.

6. A macrolide of formula (I) or a physiologically acceptable salt thereof as claimed in any one of claims

1 to 5 for use as an antibiotic in the chemotherapy of warm blooded animals.

7. A feed premix comprising as an active ingredient a macrolide of formula (I) or a physiologically acceptable salt thereof as claimed in any one of claims 1 to 5.

8. A veterinary formulation which comprises as an active ingredient a macrolide of formula (I) or a physiologically acceptable salt thereof, as claimed in any one of claims 1 to 5, associated with one or more physiologically-acceptable carriers or vehicles therefor.

**Claims for the Contracting State: AT**

1. A process for preparing a macrolide of formula (I)

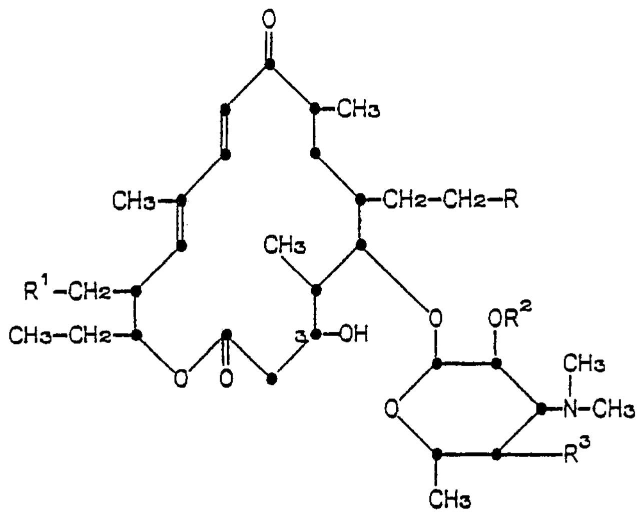

(I)

wherein

R is hydrogen, iodo, bromo, chloro, fluoro, cyano, —$OR^4$, —OAr, —$SR^5$, azido, —$NR^6R^7$, N-phthalimido or $R^9$;

$R^1$ is i) hydrogen or —OH; ii) chloro, fluoro, bromo, iodo —OAr, —O-tetrahydrofuranyl, —O-tetrahydropyranyl, —$SR^5$, azido, —$NR^6R^7$, or N-phthalimido; or $R^9$;

$R^9$ is i) a monocyclic amino group of the formula —$N(CH_2)_n$ which is optionally substituted at one or more of the carbon atoms by a $C_1$—$C_3$-alkyl, hydroxyl, methoxyl, ethoxyl, —$N(R^8)_2$,

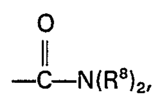

carbomethoxy, carboethoxy, or phenyl group; and n is an integer from 4 through 15; ii) a monocyclic saturated or unsaturated nitrogen-containing heterocyclic ring bonded through the nitrogen atom, said ring having 1) from 5 to 7 ring atoms which include up to 3 additional heteroatoms selected from nitrogen, oxygen and sulfur, and 2) up to 3 substituent groups selected from methyl, ethyl and phenyl; or iii) a bicyclic or tricyclic secondary amino group selected from 1,2,3,4-tetrahydroquinolin-1-yl; decahydroquinolin-1-yl; 1,2,3,4-tetrahydroisoquinolin-2-yl; decahydroisoquinolin-2-yl; indolin-1-yl; isoindolin-2-yl; decahydrocyclohepta[b]-pyrrol-1-yl; decahydrocyclohepta[c]-pyrrol-2-yl; decahydrocyclopent[c]-azepin-2-yl; decahydrocyclopent[d]azepin-3-yl; 2,3,4,5-tetrahydro-1H-2-benzazepin-2-yl) 2,3,4,5-tetrahydro-1H-3-benzazepin-3-yl; azabicycloheptanyl; azabicyclooctanyl; azabicyclononanyl; azabicyclodecanyl or azatricyclodecanyl;

$R^2$ is hydrogen, optionally substituted $C_1$—$C_5$-alkanoyl or optionally substituted benzoyl, phenylacetyl or phenylpropionyl;

$R^3$ is hydrogen, hydroxyl, optionally substituted $C_1$—$C_5$-alkanoyloxy or optionally substituted benzoyloxy, phenylacetoxy or phenylpropionyloxy or

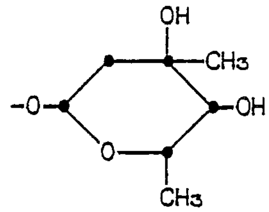

(mycarosyloxy)

$R^4$ is hydrogen, optionally substituted $C_1$—$C_4$-alkyl, cyclohexyl, optionally substituted benzyl, phenethyl or phenoxyethyl;

Ar is i) phenyl, derivatized phenyl, or naphthyl; ii) an optionally substituted heteroaryl group selected from pyridinyl, pyrimidinyl, pyridazinyl, pyrazinyl, triazinyl, indolyl, isoquinolinyl, quinolinyl, quinazolinyl, cinnolinyl, quinoxalinyl, phthalazinyl, benzotriazolyl, benzoxazolyl, benzimidazolyl, carbazolyl, or acridinyl; or

iii) optionally substituted $C_1$—$C_5$-alkanoyl; optionally substituted benzoyl, phenylacetyl, phenylpropionyl, phenoxyacetyl or phenylthioacetyl; methanesulfonyl; trifluoromethanesulfonyl; or optionally substituted phenylsulfonyl;

$R^5$ is optionally substituted $C_1$—$C_4$-alkyl; cyclohexyl; optionally substituted phenyl, benzyl or phenethyl; or an optionally substituted heteroaryl group selected from imidazolyl, pyrazolyl, pyridinyl, pyrimidinyl, pyrazinyl, pyridazinyl, triazinyl, triazolyl, tetrazolyl, oxazolyl, isoxazolyl, oxadiazolyl, thiazolyl, isothiazolyl, thiadiazolyl, thienyl and furanyl;

$R^6$ is hydrogen, optionally substituted $C_1$—$C_6$-alkyl, phenyl, benzyl, phenethyl or $C_3$—$C_8$-cycloalkyl;

$R^7$ is an $R^6$ group or optionally substituted $C_1$—$C_5$-alkanoyl, optionally substituted benzoyl, phenylacetyl, phenylpropionyl, phenoxyacetyl or phenylthioacetyl, or alkoxycarbonyl; and

$R^8$ is hydrogen, methyl, ethyl, n-propyl or isopropyl or the $R^8$ groups taken together form a polymethylene moiety such that —$N(R^8)_2$ constitutes a cyclic amino group selected from pyrrolidinyl, piperidinyl, hexahydroazepinyl or octahydroazocinyl;

provided 1) that, when $R^4$ is hydrogen, $R^1$ cannot be hydrogen or —OH; 2) that, when R or $R^1$ is —$NHR^6$ or $R^4$ or $R^8$ is hydrogen, $R^2$ must be hydrogen, $R^3$ must be hydrogen, hydroxyl, or mycarosyloxy and Ar cannot be a type (iii) substituent; 3) that, when $R^2$ is hydrogen, $R^3$ must be hydrogen, hydroxyl or mycarosyloxy; 4) that at least one of R and $R^1$ is $R^9$; 5) that when R, is H or OH, $R^9$ is not a monocyclic saturated or unsaturated nitrogen-containing heterocyclic ring as defined under ii), or a physiologically acceptable salt thereof which comprises:

(a) reducing a starting macrolide of formula (II) wherein Q is formyl and $Q^1$ is $R^1$, provided $R^1$ is not hydroxyl, to give a macrolide of formula (I) wherein R is hydroxyl;

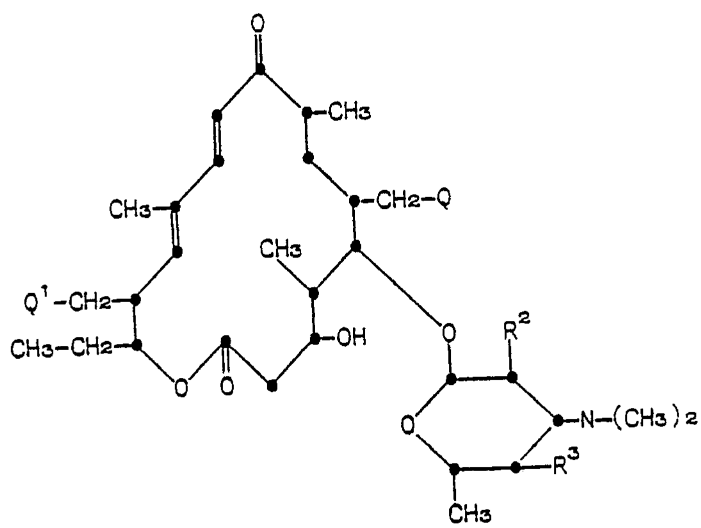

(II)

(b) reacting a starting macrolide of formula (II) wherein Q is formyl and $Q^1$ is $R^1$ with an amine of the formula $HNR^6R^6$ or $HR^9$ in the presence of a reducing agent to give a macrolide of formula (I) wherein R is $NR^6R^6$ or $R^9$, or

(c) reacting a starting macrolide of formula (II) wherein Q is —$CH_2OH$ and $Q^1$ is $R^1$ with diethylazodicarboxylate or dimethylazodicarboxylate, triphenylphosphine, and a reagent selected from

(i) an azide transfer agent, to give a macrolide of formula (I) wherein R is azido,

(ii) phthalimide to give a macrolide of formula (I) wherein R is phthalimido,

(iii) a phenol of formula ArOH to give a macrolide of formula (I) wherein R is —OAr, where Ar is a category 1) or ii) Ar group,

(iv) an alkyl halide or polyhalide to give a macrolide of formula (I) wherein R is Cl, Br, or I, or

(v) a mercaptan of formula $HSR^5$ to give a macrolide of formula (I) wherein R is $SR^5$, or

(vi) a carboxylic or sulfonic acid of the formula ArOH, where Ar is a category (iii) Ar group, to give a macrolide of formula (I) wherein R is OAr,

(d) reacting a starting macrolide of formula (II) wherein Q is $—CH_2OH$ and $Q^1$ is $R^1$ with triphenylphosphine and a halogen source to give a macrolide of formula (I) wherein R is Cl, Br, or I,

(e) reacting a starting material of formula (II) wherein Q is $—CH_2OH$ and $Q^1$ is $R^1$ with an acylating agent derived from a carboxylic or sulfonic acid of formula ArOH, where Ar is a category (iii) Ar group to give a macrolide of formula (I) wherein R is OAr, or

(f) reacting a starting macrolide of formula (II) wherein Q is $—CH_2L$ where L is a leaving group and $Q^1$ is $R^1$ or a leaving group with

(i) an alkali metal azide or halide or a tetraalkylammonium azide or fluoride where alkyl is methyl, ethyl, propyl or butyl, to give a macrolide of formula (I) wherein R is azido, F, Cl, Br, or I, or

(ii) a mercaptide ion of formula $R^5S—$ to give a macrolide of formula (I) wherein R is $R^5S—$, or

(iii) an amine of the formula $NR^6R^6$ or $HR^9$ to give a macrolide of formula (I) wherein R is $NR^6R^6$ or $R^9$, or

(iv) a source of cyanide ion to give a macrolide of formula (I) wherein R is —CN;

(v) an alcohol of the formula $HOR^4$ and a source of silver ion to provide a macrolide of formula (I) wherein R is $OR^4$ where $R^4$ is other than hydrogen

(g) hydrolyzing a macrolide of formula (II) wherein Q is $CH_2R$ and $Q^1$ is

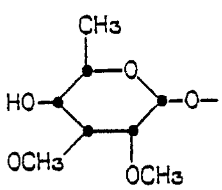 , 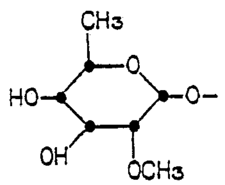 , or 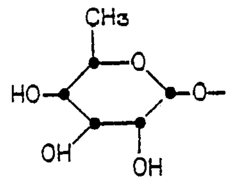

to give a macrolide of formula (I) wherein $R^1$ is hydroxyl, or

(h) removing the hydroxy protecting group from a macrolide of formula (II) wherein Q is $—CH_2R$ and $Q^1$ is protected hydroxy or

(i) reacting a starting macrolide of formula (II) wherein Q is $—CH_2R$ and $Q^1$ is hydroxyl with diethylazodicarboxylate or dimethylazodicarboxylate, triphenylphosphine, and a reagent selected from

(i) an azide transfer agent to give a macrolide of formula (I) wherein $R^1$ is azido,

(ii) phthalimide to give a macrolide of formula (I) wherein $R^1$ is phthalimido,

(iii) a phenol of formula ArOH where Ar is a category (i) or (ii) Ar group to give a macrolide of formula (I) wherein $R^1$ is —OAr,

(iv) an alkyl halide or polyhalide to give a macrolide of formula (I) where $R^1$ is Cl, Br, or I, or

(v) a mercaptan of formula $HSR^5$ to give a macrolide of formula (I) wherein R is $SR^5$, or

(vi) a carboxylic or sulfonic acid of the formula ArOH, where Ar is a category (i) Ar group, to give a macrolide of formula (I) wherein $R^1$ is OAr; or

(j) reacting a macrolide of formula (II) wherein Q is $—CH_2R$ and $Q^1$ is hydroxyl with an acylating agent derived from a carboxylic or sulfonic acid of formula ArOH where Ar is a category (iii) Ar group to give a macrolide of formula (I) wherein $R^1$ is OAr or

(k) reacting a macrolide of formula (II) wherein Q is R and $Q^1$ is hydroxyl with triphenylphosphine and a halogenating agent to give a macrolide of formula (I) wherein $R^1$ is Cl, Br or I, or

(l) reacting a macrolide of formula (II) wherein Q is $—CH_2R$ and $Q^1$ is a leaving group with

(i) an alkali metal azide or halide or a tetraalkylammonium azide or fluoride where alkyl is methyl, ethyl, propyl, or methyl to give a macrolide of formula (I) wherein $R^1$ is azido, F, Cl, Br, or I,

(ii) a mercaptide ion of formula $R^5S—$ to give a macrolide of formula I wherein $R^1$ is $R^5S—$, or

(iii) an amine of the formula $HNR^6R^6$ or $HR^9$ to give a macrolide of formula (I) wherein $R^1$ is $HR^6R^6$ or $R^9$, or

(m) reducing a macrolide of formula (II) wherein Q is $—CH_2N_3$ and $Q^1$ is $R^1$ or Q is R and $Q^1$ is azido to give a macrolide of formula (I) wherein R is $—CH_2NH_2$ or $R^1$ is amino, or

(n) acylating a macrolide of formula (II) wherein Q is $—CH_2NHR^6$ or $R^1$ is $NHR^6$ to give a macrolide of formula (I) wherein R is $—CH^2NHR^7$ or $R^1$ is $—NR^6R^7$, or

(o) esterifying a macrolide of formula (I), or

(p) salifying a macrolide of formula (I), or

(q) hydrolyzing a macrolide of formula (I) wherein $R^3$ is mycarosyloxy in acid solution at a pH below 4 to give a macrolide of formula (I) wherein $R^3$ is hydroxy, or

(r) deoxygenating a macrolide of formula (I) wherein $R^3$ is hydroxy to give a macrolide of formula (I) wherein $R^3$ is hydrogen.

(s) reacting a macrolide of formula (II) wherein Q is $CH_2I$ and $Q^1$ is $R^1$ with a reducing agent to give a

compound of formula (I) wherein R is hydrogen, or

(t) reacting a macrolide of formula (II) wherein Q is $-CH_2-$ sulfonate and $Q^1$ is $R^1$ with a source of iodide ion to give a macrolide of formula (I) wherein R is iodo.

2. A process according to claim 1 for preparing a macrolide of formula (I) wherein R is $R^9$.

3. A process according to claim 1 for preparing a macrolide of formula (I) wherein R is hydrogen, hydroxy, phenoxy, N-phthalimido, phenylacetoxy, 3-azabicyclo(3.2.2.)-nonan-3-yl, morpholino, 4-phenylpiperidin-1-yl, or octahydroazocin-1-yl.

4. A process according to claim 1 for preparing a macrolide of formula (I) wherein $R^1$ is hydrogen, hydroxy, phenoxy, acetoxy, phenylacetoxy, N-phthalimido, phenylpropionyloxy, phenylthio, octahydroazocin-1-yl, 4-hydroxypiperidino, 2,3-dimethoxyphenoxy, 3-pyridyloxy, or m-dimethylaminophenoxy.

5. A macrolide of formula (I) or a physiologically-acceptable salt thereof, as defined in any one of claims 1 to 4, for use in the suppression or treatment of bacterial or mycoplasma infections in non-human warm-blooded animals.

**Patentansprüche für die Vertragsstaaten: BE CH DE FR GB IT LI LU NL SE**

1. Makrolid der Formel (I)

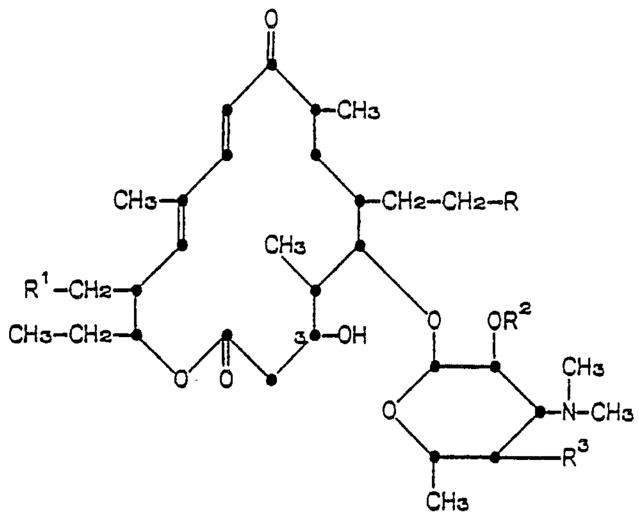

(I)

worin

R Wasserstoff, Iod, Brom, Chlor, Fluor, Cyano, $-OR^4$, $-OAr$, $-SR^5$, Azido, $-NR^6R^7$, N-Phthalimido oder $R^9$ ist,

$R^1$ für i) Wasserstoff oder $-OH$ oder ii) Chlor, Fluor, Brom, Iod, $-OAr$, -O-Tetrahydrofuranyl, -O-Tetrahydropyranyl, $-SR^5$, Azido, $-NR^6R^7$, N-Phthalimido oder $R^9$ steht,

$R^9$ für i) eine monocyclische Aminogruppe der Formel $-N(CH_2)_n$, die gegebenenfalls an einem oder mehreren ihrer Kohlenstoffatome durch eine $C_1-C_3$-Alkyl-, Hydroxyl-, Methoxyl-, Ethoxyl-, $-N(R^8)_2$,

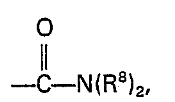

Carbomethoxy-, Carboethoxy- oder Phenylgruppe substituiert ist, worin n eine ganze Zahl von 4 bis 15 bedeutet, ii) einen über das Stickstoffatom gebundenen monocyclischen gesättigten oder ungesättigten stickstoffhaltigen heterocyclischen Ring, der (1) 5 bis 7 Ringatome aufweist und bis zu 3 weitere Heteroatome enthält, die ausgewählt sind aus Stickstoff, Sauerstoff und Schwefel, und (2) bis zu 3 Substituentengruppen aufweist, die ausgewählt sind aus Methyl, Ethyl und Phenyl, oder iii) eine bicyclische oder tricyclische sekundäre Aminogruppe steht, die ausgewählt ist aus 1,2,3,4-Tetrahydrochinolin-1-yl, Decahydrochinolin-1-yl, 1,2,3,4-Tetrahydroisochinolin-2-yl, Decahydroisochinolin-2-yl, Indolin-1-yl, Isoindolin-2-yl, Decahydrocyclohepta[b]pyrrol-1-yl, Decahydrocyclohepta[c]pyrrol-2-yl, Decahydrocyclopent[c]azepin-2-yl, Decahydrocyclopent[d]azepin-3-yl, 2,3,4,5-Tetrahydro-1H-2-benzazepin-2-yl, 2,3,4,5-Tetrahydro-1H-3-benzazepin-3-yl, Azabicycloheptanyl, Azabicyclooctanyl, Azabicyclononanyl, Azabicyclodecanyl oder Azatricyclodecanyl,

$R^2$ Wasserstoff, gegebenenfalls substituiertes $C_1-C_5$-Alkanoyl oder gegebenenfalls substituiertes Benzoyl, Phenylacetyl oder Phenylpropionyl ist,

$R^3$ Wasserstoff, Hydroxyl, gegebenenfalls substituiertes $C_1-C_5$-Alkanoyloxy oder gegebenenfalls substituiertes Benzoyloxy, Phenylacetoxy oder Phenylpropionyloxy oder die Gruppe

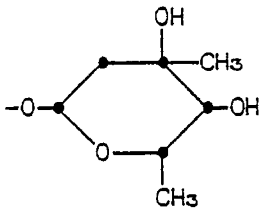

;

(mycarosyloxy)

bedeutet,

$R^4$ Wasserstoff, gegebenenfalls substituiertes $C_1$—$C_4$-Alkyl, Cyclohexyl, gegebenenfalls substituiertes Benzyl, Phenethyl oder Phenoxyethyl ist,

Ar für i) Phenyl, derivatisiertes Phenyl oder Napththyl, ii) eine gegebenenfalls substituierte Heteroarylgruppe, die ausgewählt ist aus Pyridinyl, Pyrimidinyl, Pyridazinyl, Pyrazinyl, Triazinyl, Indolyl, Isochinolinyl, Chinolinyl, Chinazolinyl, Cinnolinyl, Chinoxalinyl, Phthalazinyl, Benzotriazolyl, Benzoxazolyl, Benzimidazolyl, Carbazolyl oder Acridinyl, oder iii) gegebenenfalls substituiertes $C_1$—$C_5$-Alkanoyl, gegebenenfalls substituiertes Benzoyl, Phenylacetyl, Phenylpropionyl, Phenoxyacetyl oder Phenylthioacetyl, Methansulfonyl, Trifluormethansulfonyl oder gegebenenfalls substituiertes Phenylsulfonyl steht,

$R^5$ gegebenenfalls substituiertes $C_1$—$C_4$-Alkyl, Cyclohexyl, gegebenenfalls substituiertes Phenyl, Benzyl oder Phenethyl oder eine gegebenenfalls substituierte Heteroarylgruppe bedeutet, die ausgewählt ist aus Imidazolyl, Pyrazolyl, Pyridinyl, Pyrimidinyl, Pyrazinyl, Pyridazinyl, Triazinyl, Triazolyl, Tetrazolyl, Oxazolyl, Isoxazolyl, Oxadiazolyl, Thiazolyl, Isothiazolyl, Thiadiazolyl, Thienyl und Furanyl,

$R^6$ Wasserstoff, gegebenenfalls substituiertes $C_1$—$C_6$-Alkyl, Phenyl, Benzyl, Phenethyl oder $C_3$—$C_8$-Cycloalkyl ist,

$R^7$ eine Gruppe $R^6$ oder gegebenenfalls substituiertes $C_1$—$C_5$-Alkanoyl, gegebenenfalls substituiertes Benzoyl, Phenylacetyl, Phenylpropionyl, Phenoxyacetyl oder Phenylthioacetyl oder Alkoxycarbonyl bedeutet und

$R^8$ Wasserstoff, Methyl, Ethyl, n-Propyl oder Isopropyl ist oder die Gruppen $R^8$ zusammengenommen einen Polymethylenrest der Formel —$N(R^8)_2$ bilden, welcher eine aus Pyrrolidinyl, Piperidinyl, Hexahydroazepinyl oder Octahydroazocinyl ausgewählte cyclische Aminogruppe darstellt,

mit der Maßgabe, daß

1) $R^1$ nicht Wasserstoff oder —OH sein kann, falls $R^4$ Wasserstoff ist,

2) $R^2$ Wasserstoff sein muß, $R^3$ Wasserstoff, Hydroxyl oder Mycarosyloxy sein muß und Ar kein Substituent der oben unter iii) angegebenen Art sein kann, falls R oder $R^1$ für —$NHR^6$ steht oder $R^4$ oder $R^8$ Wasserstoff ist,

3) $R^3$ Wasserstoff, Hydroxyl oder Mycarosyloxy sein muß, falls $R^2$ Wasserstoff ist,

4) wenigstens einer der Substituenten R und $R^1$ für $R^9$ steht und

5) $R^9$ kein monocyclischer gesättigter oder ungesättigter stickstoffhaltiger heterocyclischer Ring der unter ii) angegebenen Definition sein kann, falls $R^1$ Wasserstoff oder Hydroxyl ist,

oder ein physiologisch annehmbares Salz einer solchen Verbindung.

2. Makrolid der Formel (I) nach Anspruch 1, dadurch gekennzeichnet, daß R für $R^9$ steht.

3. Makrolid der Formel (I) nach Anspruch 1, dadurch gekennzeichnet, daß R Wasserstoff, Hydroxy, Phenoxy, N-Phthalimido, Phenylacetoxy, 3-Azabicyclo(3.2.2)nonan-3-yl, Morpholino, 4-Phenylpiperidin-1-yl oder Octahydroazocin-1-yl ist.

4. Makrolid der Formel (I) nach Anspruch 1, dadurch gekennzeichnet, daß $R^1$ Wasserstoff, Hydroxy, Phenoxy, Acetoxy, Phenylacetoxy, N-Phthalimido, Phenylpropionyloxy, Phenylthio, Octahydroazocin-1-yl, 4-Hydroxypiperidino, 2,3-Dimethoxyphenoxy, 3-Pyridyloxy oder m-Dimethylaminophenoxy ist.

5. Verfahren zur Herstellung eines Makrolids der Formel (I) gemäß Definition von Anspruch 1 oder eines physiologisch annehmbaren Salzes einer solchen Verbindung, dadurch gekennzeichnet, daß man

(a) ein Ausgangsmakrolid der Formel (II)

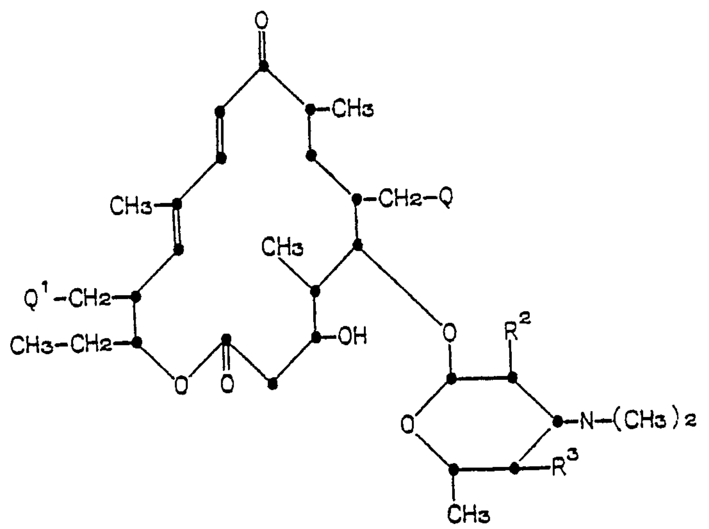

(II)

worin Q Formyl ist und $Q^1$ für $R^1$ steht, wobei $R^1$ jedoch nicht Hydroxyl sein kann, durch Reduktion in ein Makrolid der Formel (I) überführt, worin R Hydroxyl ist,

(b) ein Ausgangsmakrolid der Formel (II), worin Q Formyl ist und $Q^1$ für $R^1$ steht, durch Umsetzung mit einem Amin der Formel $HNR^6R^6$ oder $HR^9$ in Anwesenheit eines Reduktionsmittels in ein Makrolid der Formel (I) überführt, worin R für $NR^6R^6$ oder $R^9$ steht,

(c) ein Ausgangsmakrolid der Formel (II), worin Q für $—CH_2OH$ steht und $Q^1$ für $R^1$ steht, durch Umsetzung mit Diethylazodicarboxylat oder Dimethylazodicarboxylat, Triphenylphosphin und einem Reagenz, das ausgewählt ist aus

(i) einem Azidtransfermittel in ein Makrolid der Formel (I) überführt, worin R Azido ist,

(ii) Phthalimid in ein Makrolid der Formel (I) überführt, worin R Phthalimido bedeutet,

(iii) einem Phenol der Formel ArOH in ein Makrolid der Formel (I) überführt, worin R für —OAr steht, wobei Ar eine Arylgruppe der oben erwähnten Kategorie 1) oder (ii) ist,

(iv) einem Alkylhalogenid oder Polyhalogenid in ein Makrolid der Formel (I) überführt, worin R für Cl, Br oder I steht,

(v) einem Mercaptan der Formel $HSR^5$ in ein Makrolid der Formel (I) überführt, worin R für $SR^5$ steht,

(vi) einer Carbonsäure oder Sulfonsäure der Formel ArOH, worin Ar eine Arylgruppe der oben erwähnten Kategorie (iii) ist, in ein Makrolid der Formel (I) überführt, worin R für OAr steht,

(d) ein Ausgangsmakrolid der Formel (II), worin Q für $—CH_2OH$ steht und $Q^1$ für $R^1$ steht, durch Umsetzung mit Triphenylphosphin und einer Halogenquelle in ein Makrolid der Formel (I) überführt, worin R für Cl, Br oder I steht,

(e) ein Ausgangsmaterial der Formel (II), worin Q für $—CH_2OH$ steht und $Q^1$ für $R^1$ steht, durch Umsetzung mit einem Acylierungsmittel, das abgeleitet ist von einer Carbonsäure oder Sulfonsäure der Formel ArOH, worin Ar eine Arylgruppe der oben erwähnten Kategorie (iii) ist, in ein Makrolid der Formel (I) überführt, worin R für OAr steht,

(f) ein Ausgangsmakrolid der Formel (II), worin Q für $—CH_2L$ steht, worin L eine Abgangsgruppe ist, und $Q^1$ für $R^1$ oder eine Abgangsgruppe steht, durch Umsetzung mit

(i) einem Alkalimetallazid oder Alkalimetallhalogenid oder einem Tetraalkylammoniumazid oder Tetraalkylammoniumfluorid, worin Alkyl für Methyl, Ethyl, Propyl oder Butyl steht, in ein Makrolid der Formel (I) überführt, worin R Azido, F, Cl, Br oder I ist,

(ii) einem Mercaptidion der Formel $R^5S—$ in ein Makrolid der Formel (I) überführt, worin R für $R^5S—$ steht,

(iii) einem Amin der Formel $NR^6R^6$ oder $HR^9$ in ein Makrolid der Formel (I) überführt, worin R für $NR^6R^6$ oder $R^9$ steht,

(iv) einer Quelle für Cyanidionen in ein Makrolid der Formel (I) überführt, worin R für —CN steht,

(v) einem Alkohol der Formel $HOR^4$ und einer Quelle für Silberionen in ein Makrolid der Formel (I) überführt, worin R für $OR^4$ steht, wobei $R^4$ etwas anderes als Wasserstoff ist,

(g) ein Makrolid der Formel (II), worin Q für $CH_2R$ steht und $Q^1$ einen der folgenden Reste bedeutet

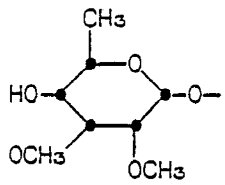 , 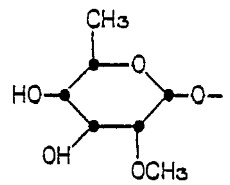 oder 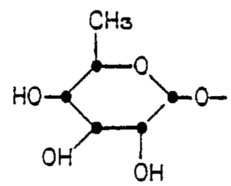

durch Hydrolyse in ein Makrolid der Formel (I) überführt, worin $R^1$ Hydroxyl ist,

(h) von einem Makrolid der Formel (II), worin Q für $—CH_2R$ steht und $Q^1$ geschütztes Hydroxy ist, die Hydroxyschutzgruppe entfernt,

(i) ein Ausgangsmakrolid der Formel (II), worin Q für $—CH_2R$ steht und $Q^1$ Hydroxyl ist, durch Umsetzung mit Diethylazodicarboxylat oder Dimethylazodicarboxylat, Triphenylphosphin und einem Reagenz, das ausgewählt ist aus

(i) einem Azidtransfermittel in ein Makrolid der Formel (I) überführt, worin $R^1$ Azido ist,

(ii) Phthalimid in ein Makrolid der Formel (I) überführt, worin $R^1$ Phthalimido ist,

(iii) einem Phenol der Formel ArOH, worin Ar eine Arylgruppe der oben erwähnten Kategorie (i) oder (ii) ist, in ein Makrolid der Formel (I) überführt, worin $R^1$ für —OAr steht,

(iv) einem Alkylhalogenid oder Polyhalogenid in ein Makrolid der Formel (I) überführt, worin $R^1$ für Cl, Br oder I steht,

(v) einem Mercaptan der Formel $HSR^5$ in ein Makrolid der Formel (I) überführt, worin R für $SR^5$ steht,

(vi) einer Carbonsäure oder Sulfonsäure der Formel ArOH, worin Ar eine Arylgruppe der oben erwähnten Kategorie (i) ist, in ein Makrolid der Formel (I) überführt, worin $R^1$ für OAr steht,

(j) ein Makrolid der Formel (II), worin Q für $—CH_2R$ steht und $Q^1$ Hydroxyl ist, durch Umsetzung mit einem Acylierungsmittel, das von einer Carbonsäure oder Sulfonsäure der Formel ArOH abgeleitet ist, worin Ar eine Arylgruppe der oben erwähnten Kategorie (iii) ist, in ein Makrolid der Formel (I) überführt, worin $R^1$ für OAr steht,

(k) ein Makrolid der Formel (II), worin Q für R steht und $Q^1$ Hydroxyl ist, durch Umsetzung mit Triphenylphosphin und einem Halogenierungsmittel in ein Makrolid der Formel (I) überführt, worin $R^1$ für Cl, Br oder I steht,

(l) ein Makrolid der Formel (II), worin Q für $—CH_2R$ steht und $Q^1$ eine Abgangsgruppe ist, durch Umsetzung mit

(i) einem Alkalimetallazid oder Alkalimetallhalogenid oder einem Tetraalkylammoniumazid oder Tetraalkylammoniumfluorid, worin Alkyl für Methyl, Ethyl, Propyl oder Butyl steht, in ein Makrolid der Formel (I) überführt, worin $R^1$ Azido, F, Cl, Br oder I ist,

(ii) einem Mercaptidion der Formel $R^5S—$ in ein Makrolid der Formel (I) überführt, worin $R^1$ für $R^5S—$ steht,

(iii) einem Amin der Formel $HNR^6R^6$ oder $HR^9$ in ein Makrolid der Formel (I) überführt, worin $R^1$ für $HR^6R^6$ oder $R^9$ steht,

(m) ein Makrolid der Formel (II), worin Q für $—CH_2N_3$ steht und $Q^1$ für $R^1$ steht oder Q für R steht und $Q^1$ Azido ist, durch Reduktion in ein Makrolid der Formel (I) überführt, worin R für $—CH_2NH_2$ steht oder $R^1$ Amino ist,

(n) ein Makrolid der Formel (II), worin Q für $—CH_2NHR^6$ steht oder $R^1$ für $NHR^6$ steht, durch Acylierung in ein Makrolid der Formel (I) überführt, worin R für $—CH^2NHR^7$ steht oder $R^1$ für $—NR^6R^7$ steht,

(o) ein Makrolid der Formel (I) einer Veresterung unterzieht,

(p) ein Makrolid der Formel (I) einer Salzbildung unterzieht,

(q) ein Makrolid der Formel (I), worin $R^3$ Mycarosyloxy ist, durch Hydrolyse in einer sauren Lösung bei einem pH-Wert von unter 4 in ein Makrolid der Formel (I) überführt, worin $R^3$ Hydroxy ist,

(r) ein Makrolid der Formel (I), worin $R^3$ Hydroxy ist, durch Deoxygenierung in ein Makrolid der Formel (I) überführt, worin $R^3$ Wasserstoff ist,

(s) ein Makrolid der Formel (II), worin Q für $CH_2I$ steht und $Q^1$ für $R^1$ steht, durch Umsetzung mit einem Reduktionsmittel in eine Verbindung der Formel (I) überführt, worin R Wasserstoff ist, oder

(t) ein Makrolid der Formel (II), worin Q für $—CH_2$-Sulfonat steht und $Q^1$ für $R^1$ steht, durch Umsetzung mit einer Quelle für Iodidionen in ein Makrolid der Formel (I) überführt, worin R Iod ist.

6. Verwendung eines Makrolids der Formel (I) oder eines physiologisch annehmbaren Salzes hiervon nach irgendeinem der Ansprüche 1 bis 5 als Antibiotikum bei der chemotherapeutischen Behandlung warmblütiger Tiere.

7. Futtervormischung, dadurch gekennzeichnet, daß sie als Wirkstoff ein Makrolid der Formel (I) oder ein physiologisch annehmbares Salz hiervon nach irgendeinem der Ansprüche 1 bis 5 enthält.

8. Veterinärformulierung, dadurch gekennzeichnet, daß sie ein Makrolid der Formel (I) oder ein physiologisch annehmbares Salz hiervon nach irgendeinem der Ansprüche 1 bis 5 als Wirkstoff in Kombination mit einem oder mehreren physiologisch annehmbaren Trägern oder Schleppmitteln hierfür enthält.

**Patentansprüche für den Vertragsstaat: AT**

1. Verfahren zur Herstellung eines Makrolids der Formel (I)

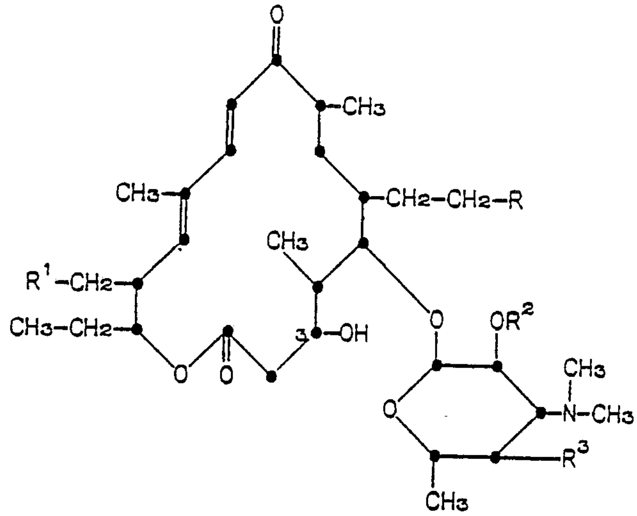

(I)

worin

R Wasserstoff, Iod, Brom, Chlor, Fluor, Cyano, —$OR^4$, —OAr, —$SR^5$, Azido, —$NR^6R^7$, N-Phthalimido oder $R^9$ ist,

$R^1$ für i) Wasserstoff oder —OH oder ii) Chlor, Fluor, Brom, Iod, —OAr, —O-Tetrahydrofuranyl, —O-Tetrahydropyranyl, —$SR^5$, Azido, —$NR^6R^7$, N-Phthalimido oder $R^9$ steht,

$R^9$ für i) eine monocyclische Aminogruppe der Formel —$N(CH_2)_n$, die gegebenenfalls an einem oder mehreren ihrer Kohlenstoffatome durch eine $C_1$—$C_3$-Alkyl-, Hydroxyl-, Methoxyl-, Ethoxyl-, —$N(R^8)_2$,

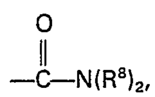

Carbomethoxy-, Carboethoxy- oder Phenylgruppe substituiert ist, worin n eine ganze Zahl von 4 bis 15 bedeutet, ii) einen über das Stickstoffatom gebundenen monocyclischen gesättigten oder ungesättigten stickstoffhaltigen heterocyclischen Ring, der (1) 5 bis 7 Ringatome aufweist und bis zu 3 weitere Heteroatome enthält, die ausgewählt sind aus Stickstoff, Sauerstoff und Schwefel, und (2) bis zu 3 Substituentengruppen aufweist, die ausgewählt sind aus Methyl, Ethyl und Phenyl, oder iii) eine bicyclische oder tricyclische sekundäre Aminogruppe steht, die ausgewählt ist aus 1,2,3,4-Tetrahydrochinolin-1-yl, Decahydrochinolin-1-yl, 1,2,3,4-Tetrahydroisochinolin-2-yl, Decahydroisochinolin-2-yl, Indolin-1-yl, Isoindolin-2-yl, Decahydrocyclohepta[b]pyrrol-1-yl, Decahydrocyclohepta[c]pyrrol-2-yl, Decahydrocyclopent[c]azepin-2-yl, Decahydrocyclopent[d]azepin-3-yl, 2,3,4,5-Tetrahydro-1H-2-benzazepin-2-yl, 2,3,4,5-Tetrahydro-1H-3-benzazepin-3-yl, Azabicycloheptanyl, Azabicyclooctanyl, Azabicyclononanyl, Azabicyclodecanyl oder Azatricyclodecanyl,

$R^2$ Wasserstoff, gegebenenfalls substituiertes $C_1$—$C_5$-Alkanoyl oder gegebenenfalls substituiertes Benzoyl, Phenylacetyl oder Phenylpropionyl ist,

$R^3$ Wasserstoff, Hydroxyl, gegebenenfalls substituiertes $C_1$—$C_5$-Alkanoyloxy oder gegebenenfalls substituiertes Benzoyloxy, Phenylacetoxy oder Phenylpropionyloxy oder die Gruppe

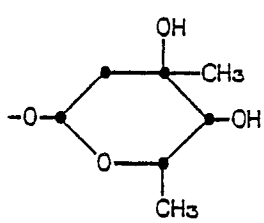

;

(mycarosyloxy)

bedeutet,

$R^4$ Wasserstoff, gegebenenfalls substituiertes $C_1$—$C_4$-Alkyl, Cyclohexyl, gegebenenfalls substituiertes Benzyl, Phenethyl oder Phenoxyethyl ist,

Ar für i) Phenyl, derivatisiertes Phenyl oder Napththyl, ii) eine gegebenenfalls substituierte Heteroarylgruppe, die ausgewählt ist aus Pyridinyl, Pyrimidinyl, Pyridazinyl, Pyrazinyl, Triazinyl, Indolyl, Isochinolinyl, Chinolinyl, Chinazolinyl, Cinnolinyl, Chinoxalinyl, Phthalazinyl, Benzotriazolyl, Benzoxazolyl, Benzimidazolyl, Carbazolyl oder Acridinyl, oder iii) gegebenenfalls substituiertes $C_1$—$C_5$-Alkanoyl, gegebenenfalls substituiertes Benzoyl, Phenylacetyl, Phenylpropionyl, Phenoxyacetyl oder Phenylthioacetyl, Methansulfonyl, Trifluormethansulfonyl oder gegebenenfalls substituiertes Phenylsulfonyl steht,

$R^5$ gegebenenfalls substituiertes $C_1$—$C_4$-Alkyl, Cyclohexyl, gegebenenfalls substituiertes Phenyl, Benzyl oder Phenethyl oder eine gegebenenfalls substituierte Heteroarylgruppe bedeutet, die ausgewählt ist aus Imidazolyl, Pyrazolyl, Pyridinyl, Pyrimidinyl, Pyrazinyl, Pyridazinyl, Triazinyl, Triazolyl, Tetrazolyl, Oxazolyl, Isoxazolyl, Oxadiazolyl, Thiazolyl, Isothiazolyl, Thiadiazolyl, Thienyl und Furanyl,

$R^6$ Wasserstoff, gegebenenfalls substituiertes $C_1$—$C_6$-Alkyl, Phenyl, Benzyl, Phenethyl oder $C_3$—$C_8$-Cycloalkyl ist,

$R^7$ eine Gruppe $R^6$ oder gegebenenfalls substituiertes $C_1$—$C_5$-Alkanoyl, gegebenenfalls substituiertes Benzoyl, Phenylacetyl, Phenylpropionyl, Phenoxyacetyl oder Phenylthioacetyl oder Alkoxycarbonyl bedeutet und

$R^8$ Wasserstoff, Methyl, Ethyl, n-Propyl oder Isopropyl ist oder die Gruppen $R^8$ zusammengenommen einen Polymethylenrest der Formel —$N(R^8)_2$ bilden, welcher eine aus Pyrrolidinyl, Piperidinyl, Hexahydroazepinyl oder Octahydroazocinyl ausgewählte cyclische Aminogruppe darstellt,

mit der Maßgabe, daß

1) $R^1$ nicht Wasserstoff oder —OH sein kann, falls $R^4$ Wasserstoff ist,

2) $R^2$ Wasserstoff sein muß, $R^3$ Wasserstoff, Hydroxyl oder Mycarosyloxy sein muß und Ar kein Substituent der oben unter iii) angegebenen Art sein kann, falls R oder $R^1$ für —$NHR^6$ steht oder $R^4$ oder $R^8$ Wasserstoff ist,

3) $R^3$ Wasserstoff, Hydroxyl oder Mycarosyloxy sein muß, falls $R^2$ Wasserstoff ist,

4) wenigstens einer der Substituenten R und $R^1$ für $R^9$ steht und

5) $R^9$ kein monocyclischer gesättigter oder ungesättigter stickstoffhaltiger heterocyclischer Ring der unter ii) angegebenen Definition sein kann, falls $R^1$ Wasserstoff oder Hydroxyl ist,

oder ein physiologisch annehmbares Salz einer solchen Verbindung, dadurch gekennzeichnet, daß man

(a) ein Ausgangsmakrolid der Formel (II)

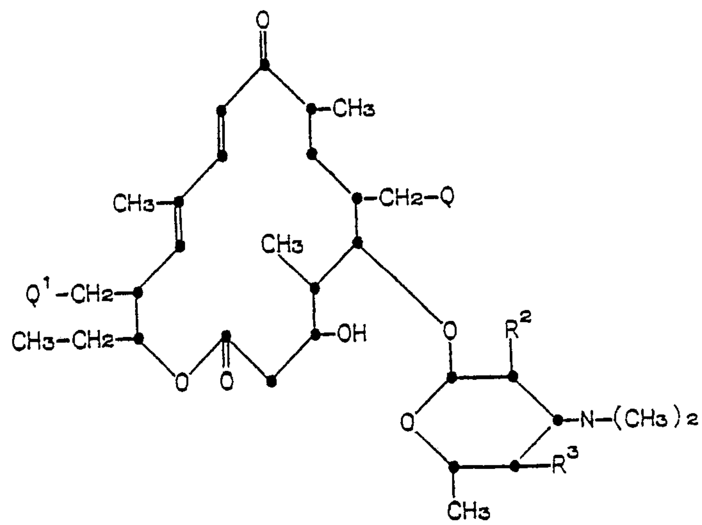

(II)

worin Q Formyl ist und $Q^1$ für $R^1$ steht, wobei $R^1$ jedoch nicht Hydroxyl sein kann, durch Reduktion in ein Makrolid der Formel (I) überführt, worin R Hydroxyl ist,

(b) ein Ausgangsmakrolid der Formel (II), worin Q Formyl ist und $Q^1$ für $R^1$ steht, durch Umsetzung mit einem Amin der Formel $HNR^6R^6$ oder $HR^9$ in Anwesenheit eines Reduktionsmittels in ein Makrolid der Formel (I) überführt, worin R für $NR^6R^6$ oder $R^9$ steht,

(c) ein Ausgangsmakrolid der Formel (II), worin Q für —$CH_2OH$ steht und $Q^1$ für $R^1$ steht, durch Umsetzung mit Diethylazodicarboxylat oder Dimethylazodicarboxylat, Triphenylphosphin und einem Reagenz, das ausgewählt ist aus

(i) einem Azidtransfermittel in ein Makrolid der Formel (I) überführt, worin R Azido ist,

(ii) Phthalimid in ein Makrolid der Formel (I) überführt, worin R Phthalimido bedeutet,

(iii) einem Phenol der Formel ArOH in ein Makrolid der Formel (I) überführt, worin R für —OAr steht, wobei Ar eine Arylgruppe der oben erwähnten Kategorie 1) oder (ii) ist,

(iv) einem Alkylhalogenid oder Polyhalogenid in ein Makrolid der Formel (I) überführt, worin R für Cl, Br oder I steht,

(v) einem Mercaptan der Formel $HSR^5$ in ein Makrolid der Formel (I) überführt, worin R für $SR^5$ steht,

(vi) einer Carbonsäure oder Sulfonsäure der Formel ArOH, worin Ar eine Arylgruppe der oben erwähnten Kategorie (iii) ist, in ein Makrolid der Formel (I) überführt, worin R für OAr steht,

(d) ein Ausgangsmakrolid der Formel (II), worin Q für $—CH_2OH$ steht und $Q^1$ für $R^1$ steht, durch Umsetzung mit Triphenylphosphin und einer Halogenquelle in ein Makrolid der Formel (I) überführt, worin R für Cl, Br oder I steht,

(e) ein Ausgangsmaterial der Formel (II), worin Q für $—CH_2OH$ steht und $Q^1$ für $R^1$ steht, durch Umsetzung mit einem Acylierungsmittel, das abgeleitet ist von einer Carbonsäure oder Sulfonsäure der Formel ArOH, worin Ar eine Arylgruppe der oben erwähnten Kategorie (iii) ist, in ein Makrolid der Formel (I) überführt, worin R für OAr steht,

(f) ein Ausgangsmakrolid der Formel (II), worin Q für $—CH_2L$ steht, worin L eine Abgangsgruppe ist, und $Q^1$ für $R^1$ oder eine Abgangsgruppe steht, durch Umsetzung mit

(i) einem Alkalimetallazid oder Alkalimetallhalogenid oder einem Tetraalkylammoniumazid oder Tetraalkylammoniumfluorid, worin Alkyl für Methyl, Ethyl, Propyl oder Butyl steht, in ein Makrolid der Formel (I) überführt, worin R Azido, F, Cl, Br oder I ist,

(ii) einem Mercaptidion der Formel $R^5S—$ in ein Makrolid der Formel (I) überführt, worin R für $R^5S—$ steht,

(iii) einem Amin der Formel $NR^6R^6$ oder $HR^9$ in ein Makrolid der Formel (I) überführt, worin R für $NR^6R^6$ oder $R^9$ steht,

(iv) einer Quelle für Cyanidionen in ein Makrolid der Formel (I) überführt, worin R für —CN steht,

(v) einem Alkohol der Formel $HOR^4$ und einer Quelle für Silberionen in ein Makrolid der Formel (I) überführt, worin R für $OR^4$ steht, wobei $R^4$ etwas anderes als Wasserstoff ist,

(g) ein Makrolid der Formel (II), worin Q für $CH_2R$ steht und $Q^1$ einen der folgenden Reste bedeutet

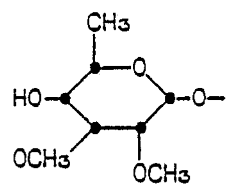 , 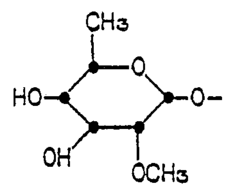 , oder 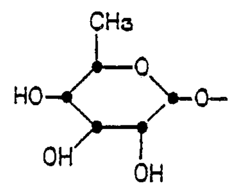

durch Hydrolyse in ein Makrolid der Formel (I) überführt, worin $R^1$ Hydroxyl ist,

(h) von einem Makrolid der Formel (II), worin Q für $—CH_2R$ steht und $Q^1$ geschütztes Hydroxy ist, die Hydroxyschutzgruppe entfernt,

(i) ein Ausgangsmakrolid der Formel (II), worin Q für $—CH_2R$ steht und $Q^1$ Hydroxyl ist, durch Umsetzung mit Diethylazodicarboxylat oder Dimethylazodicarboxylat, Triphenylphosphin und einem Reagenz, das ausgewählt ist aus

(i) einem Azidtransfermittel in ein Makrolid der Formel (I) überführt, worin $R^1$ Azido ist,

(ii) Phthalimid in ein Makrolid der Formel (I) überführt, worin $R^1$ Phthalimido ist,

(iii) einem Phenol der Formel ArOH, worin Ar eine Arylgruppe der oben erwähnten Kategorie (i) oder (ii) ist, in ein Makrolid der Formel (I) überführt, worin $R^1$ für —OAr steht,

(iv) einem Alkylhalogenid oder Polyhalogenid in ein Makrolid der Formel (I) überführt, worin $R^1$ für Cl, Br oder I steht,

(v) einem Mercaptan der Formel $HSR^5$ in ein Makrolid der Formel (I) überführt, worin R für $SR^5$ steht,

(vi) einer Carbonsäure oder Sulfonsäure der Formel ArOH, worin Ar eine Arylgruppe der oben erwähnten Kategorie (i) ist, in ein Makrolid der Formel (I) überführt, worin $R^1$ für OAr steht,

(j) ein Makrolid der Formel (II), worin Q für $—CH_2R$ steht und $Q^1$ Hydroxyl ist, durch Umsetzung mit einem Acylierungsmittel, das von einer Carbonsäure oder Sulfonsäure der Formel ArOH abgeleitet ist, worin Ar eine Arylgruppe der oben erwähnten Kategorie (iii) ist, in ein Makrolid der Formel (I) überführt, worin $R^1$ für OAr steht,

(k) ein Makrolid der Formel (II), worin Q für R steht und $Q^1$ Hydroxyl ist, durch Umsetzung mit Triphenylphosphin und einem Halogenierungsmittel in ein Makrolid der Formel (I) überführt, worin $R^1$ für Cl, Br oder I steht,

(l) ein Makrolid der Formel (II), worin Q für $—CH_2R$ steht und $Q^1$ eine Abgangsgruppe ist, durch Umsetzung mit

(i) einem Alkalimetallazid oder Alkalimetallhalogenid oder einem Tetraalkylammoniumazid oder Tetraalkylammoniumfluorid, worin Alkyl für Methyl, Ethyl, Propyl oder Butyl steht, in ein Makrolid der Formel (I) überführt, worin $R^1$ Azido, F, Cl, Br oder I ist,

(ii) einem Mercaptidion der Formel $R^5S—$ in ein Makrolid der Formel (I) überführt, worin $R^1$ für $R^5S—$ steht,

(iii) einem Amin der Formel $HNR^6R^6$ oder $HR^9$ in ein Makrolid der Formel (I) überführt, worin $R^1$ für $HR^6R^6$ oder $R^9$ steht,

(m) ein Makrolid der Formel (II), worin Q für $—CH_2N_3$ steht und $Q^1$ für $R^1$ steht oder Q für R steht und $Q^1$ Azido ist, durch Reduktion in ein Makrolid der Formel (I) überführt, worin R für $—CH_2NH_2$ steht oder $R^1$ Amino ist,

(n) ein Makrolid der Formel (II), worin Q für $-CH_2NHR^6$ steht oder $R^1$ für $NHR^6$ steht, durch Acylierung in ein Makrolid der Formel (I) überführt, worin R für $-CH^2NHR^7$ steht oder $R^1$ für $-NR^6R^7$ steht,

(o) ein Makrolid der Formel (I) einer Veresterung unterzieht,

(p) ein Makrolid der Formel (I) einer Salzbildung unterzieht,

(q) ein Makrolid der Formel (I), worin $R^3$ Mycarosyloxy ist, durch Hydrolyse in einer sauren Lösung bei einem pH-Wert von unter 4 in ein Makrolid der Formel (I) überführt, worin $R^3$ Hydroxy ist,

(r) ein Makrolid der Formel (I), worin $R^3$ Hydroxy ist, durch Deoxygenierung in ein Makrolid der Formel (I) überführt, worin $R^3$ Wasserstoff ist,

(s) ein Makrolid der Formel (II), worin Q für $CH_2I$ steht und $Q^1$ für $R^1$ steht, durch Umsetzung mit einem Reduktionsmittel in eine Verbindung der Formel (I) überführt, worin R Wasserstoff ist, oder

(t) ein Makrolid der Formel (II), worin Q für $-CH_2$-Sulfonat steht und $Q^1$ für $R^1$ steht, durch Umsetzung mit einer Quelle für Iodidionen in ein Makrolid der Formel (I) überführt, worin R Iod ist.

2. Verfahren nach Anspruch 1, dadurch gekennzeichnet, daß ein Makrolid der Formel (I) hergestellt wird, worin R für $R^9$ steht.

3. Verfahren nach Anspruch 1, dadurch gekennzeichnet, daß ein Makrolid der Formel (I) hergestellt wird, worin R Wasserstoff, Hydroxy, Phenoxy, N-Phthalimido, Phenylacetoxy, 3-Azabicyclo(3.2.2)nonan-3-yl, Morpholino, 4-Phenylpiperidin-1-yl oder Octahydroazocin-1-yl ist.

4. Verfahren nach Anspruch 1, dadurch gekennzeichnet, daß ein Makrolid der Formel (I) hergestellt wird, worin $R^1$ Wasserstoff, Hydroxy, Phenoxy, Acetoxy, Phenylacetoxy, N-Phthalimido, Phenylpropionyloxy, Phenylthio, Octahydroazocin-1-yl, 4-Hydroxypiperidino, 2,3-Dimethoxyphenoxy, 3-Pyridyloxy oder m-Dimethylaminophenoxy ist.

5. Verfahren eines Makrolids der Formel (I) oder eines physiologisch annehmbaren Salzes hiervon nach irgendeinem der Ansprüche 1 bis 4 zur Unterdrückung oder Behandlung von Infektionen durch Bakterien oder Mycoplasmen bei warmblütigen Tieren mit Ausnahme des Menschen.

**Revendications pour les Etats contractants: BE CH DE FR GB IT LI LU NL SE**

1. Macrolide répondant à la formule (I):

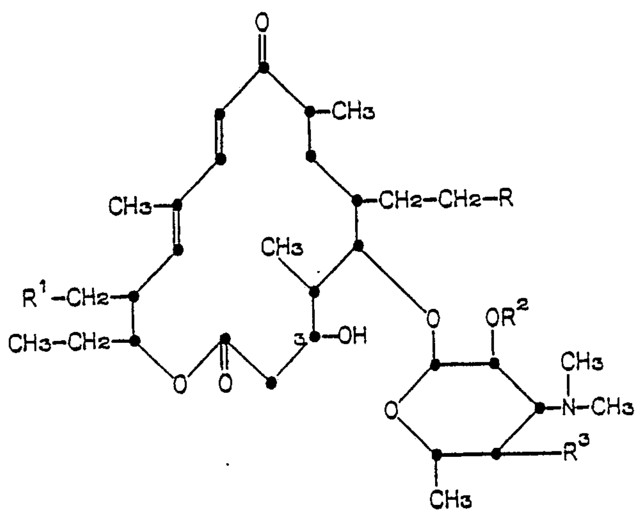

(I)

dans laquelle

R représente un atome d'hydrogène, d'iode, de brome, de chlore ou de fluor, un groupe cyano, $-OR^4$, $-OAr$, $-SR^5$, azido, $-NR^6R^7$, N-phtalimido ou $R^9$,

$R^1$ représente i) un atome d'hydrogène ou $-OH$; ii) un atome de chlore, de fluor, de brome ou d'iode, un groupe $-OAr$, $-O$-tétrahydrofurannyle, $-O$-tétrahydropyrannyle, $-SR^5$, azido, $-NR^6R^7$ ou N-phtalimido; ou encore $R^9$;

$R^9$ représente i) un groupe amino monocyclique de formule $-N(CH_2)_n$ facultativement substitué sur un ou plusieurs atomes de carbone par un groupe alkyle en $C_1$—$C_3$, un groupe hydroxyle, un groupe méthoxy, un groupe éthoxy, $-N(R^8)_2$,

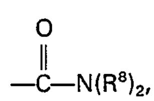

un groupe carbométhoxy, un groupe carbéthoxy ou un groupe phényle; et n est un nombre entier de 4 à 15; ii) un noyau hétérocyclique azoté monocyclique saturé ou non et lié par l'atome d'azote, ce noyau

comportant 1) 5 à 7 atomes comprenant jusqu'à 3 hétéro-atomes supplémentaires choisis parmi l'azote, l'oxygène et le soufre et 2) jusqu'à 3 substituants choisis parmi les groupes méthyle, éthyle et phényle; ou iii) un groupe sec.-amino bicyclique ou tricyclique choisi parmi les groupes 1,2,3,4-tétrahydroquinoléin-1-yle, décahydroquinoléin-1-yle, 1,2,3,4-tétrahydroisoquinoléin-2-yle, décahydroisoquinoléin-2-yle, indolin-1-yle, isoindolin-2-yle, décahydrocyclohepta[b]-pyrrol-1-yle, décahydrocyclohepta[c]-pyrrol-2-yle, décahydrocyclopent[c]-azépin-2-yle, décahydrocyclopent[d]-azépin-3-yle, 2,3,4,5-tétrahydro-1H-2-benzazépin-2-yle, 2,3,4,5-tétrahydro-1H-3-benzazépin-3-yle, azabicycloheptanyle, azabicyclo-octanyle, azabicyclononanyle, azabicyclodécanyle ou azatricyclodécanyle;

$R^2$ représente un atome d'hydrogène, un groupe alcanoyle en $C_1$—$C_5$ facultativement substitué ou un groupe phénylpropionyle, phénylacétyle ou benzoyle facultativement substitué;

$R^3$ représente un atome d'hydrogène, un groupe hydroxyle, un groupe alcanoyl(en $C_1$—$C_5$)oxy facultativement substitué ou un groupe phénylproprionyloxy, phénylacétoxy ou benzoyloxy facultativement substitué ou encore

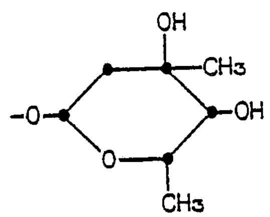 ;

(mycarosyloxy)

$R^4$ représente un atome d'hydrogène, un groupe alkyle en $C_1$—$C_4$ facultativement substitué, un groupe cyclohexyle, un groupe phénoxyéthyle, un groupe phénéthyle ou un groupe benzyle facultativement substitué;

Ar représente i) un groupe phényle, un groupe phényle à dérivation ou un groupe naphtyle; ii) un groupe hétéro-aryle facultativement substitué choisi parmi les groupes pyridinyle, pyrimidinyle, pyridazinyle, pyrazinyle, triazinyle, indolyle, isoquinoléinyle, quinoléinyle, quinazolinyle, cinnolinyle, quinoxalinyle, phtalazinyle, benzotriazolyle, benzoxazolyle, benzimidazolyle, carbazolyle ou acridinyle; ou iii) un groupe alcanoyle en $C_1$—$C_5$ facultativement substitué; un groupe phénylthio-acétyle, phénoxyacétyle, phénylpropionyle, phénylacétyle ou benzoyle facultativement substitué; un groupe méthane-sulfonyle; un groupe trifluorométhane-sulfonyle; ou un groupe phénylsulfonyle facultativement substitué;

$R^5$ représente un groupe alkyle en $C_1$—$C_4$ facultativement substitué; un groupe cyclohexyle; un groupe phénéthyle, un groupe benzyle ou un groupe phényle facultativement substitué;
ou encore un groupe hétéro-aryle facultativement substitué et choisi parmi les groupes imidazolyle, pyrazolyle, pyridinyle, pyrimidinyle, pyrazinyle, pyridazinyle, triazinyle, triazolyle, tétrazolyle, oxazolyle, isoxazolyle, oxadiazolyle, thiazolyle, isothiazolyle, thiadiazolyle, thiényle et furannyle;

$R^6$ representé un atome d'hydrogène, un groupe cycloalkyle en $C_3$—$C_8$, un groupe phénéthyle, un groupe benzyle, un groupe phényle ou un groupe alkyle en $C_1$—$C_6$ facultativement substitué;

$R^7$ représente un groupe $R^6$ ou un groupe alcanoyle en $C_1$—$C_5$ facultativement substitué, un groupe phénylthioacétyle, un groupe phénoxyacétyle, un groupe phénylpropionyle, un groupe phénylacétyle ou un groupe benzoyle facultativement substitué, ou encore un groupe alcoxycarbonyle; et

$R^8$ représente un atome d'hydrogène, un groupe méthyle, un groupe éthyle, un groupe n-propyle, ou un groupe isopropyle ou les groupes $R^8$ pris ensemble forment une fraction polyméthylène de telle sorte que —$N(R^8)_2$ constitue un groupe amino cyclique choisi parmi les groupes pyrrolidinyle, pipéridinyle, hexadro-azépinyle ou octahydro-azocinyle;
avec cette réserve que 1) si $R^4$ représente un atome d'hydrogène, $R^1$ représente un atome d'hydrogène, $R^1$ ne peut pas être un atome d'hydrogène ou —OH; 2) lorsque R ou $R^1$ représente —$NHR^6$ ou $R^4$ ou $R^8$ représente un atome d'hydrogène, $R^2$ doit être un atome d'hydrogène, $R^3$ doit être un atome d'hydrogène, un groupe hydroxyle ou un groupe mycarosyloxy et Ar ne peut pas être un substituant du type (iii); 3) lorsque $R^2$ représente un atome d'hydrogène, $R^3$ doit être un atome d'hydrogène, un groupe hydroxyle ou un groupe mycarosyloxy; 4) au moins un des radicaux R et $R^1$ doit être $R^9$; 5) lorsque $R^1$ représente H ou OH, $R^9$ ne représente pas un noyau hétérocyclique azoté monocyclique saturé ou non comme défini sub ii); ou un de ses sels physiologiquement acceptables.

2. Macrolide de formule (I) selon la revendication 1, caractérisé en ce que R est $R^9$.

3. Macrolide de formule (I) selon la revendication 1, caractérisé en ce que R représente un atome d'hydrogène, un groupe hydroxyle, un groupe phénoxy, un groupe N-phtalimido, un groupe phénylacétoxy, un groupe 3-azabicyclo(3.2.2)nonan-3-yle, un groupe morpholino, un groupe 4-phényl-pipéridin-1-yle ou un groupe octahydroazocin-1-yle.

4. Macrolide de formule (I) selon la revendication 1, caractérisé en ce que $R^1$ représente un atome d'hydrogène, un groupe hydroxyle, un groupe phénoxy, un groupe acétoxy, un groupe phénylacétoxy, un

groupe N-phtalimido, un groupe phénylpropionyloxy, un groupe phénylthio, un groupe octahydro-azocin-1-yle, un groupe 4-hydroxypipéridino, un groupe 2,3-diméthoxyphénoxy, un groupe 3-pyridyloxy ou un groupe m-diméthylaminophénoxy.

5. Procédé de préparation d'un macrolide de formule (I), comme défini dans la revendication 1, ou d'un de ses sels physiologiquement acceptables, caractérisé en ce qu'il consiste à:

(a) réduire un macrolide de départ répondant à la formule (II) dans laquelle Q représente un groupe formyle et $Q^1$ représente $R^1$, avec cette réserve que $R^1$ ne représente pas un groupe hydroxyle, pour donner un macrolide de formule (I) dans laquelle R représente un groupe hydroxyle:

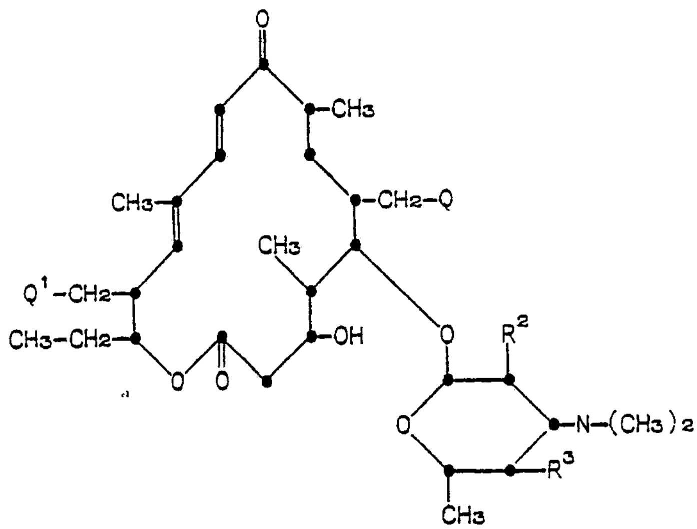

(II)

(b) faire réagir un macrolide de départ répondant à la formule (II) dans laquelle Q représente un groupe formyle et $Q^1$ représente $R^1$, avec une amine de formule $HNR^6R^6$ ou $HR^9$, en présence d'un agent réducteur, pour obtenir un macrolide de formule (I) dans laquelle R représente $NR^6R^6$ ou $R^9$; ou

(c) faire réagir un macrolide de départ répondant à la formule (II) dans laquelle Q représente —$CH_2OH$ et $Q^1$ représente $R^1$, avec un azodicarboxylate de diéthyle ou de diméthyle, de la triphénylphosphine et un réactif choisi parmi

(i) un agent de transfert d'azide, pour donner un macrolide de formule (I) dans laquelle R représente un groupe azido,

(ii) un phtalimide, pour donner un macrolide de formule (I) dans laquelle R représente un groupe phtalimido,

(iii) un groupe phénol de formule ArOH, pour donner un macrolide de formule (I) dans laquelle R représente —OAr où Ar est un groupe Ar de la catégorie i) ou ii),

(iv) un halogénure ou un polyhalogénure d'alkyle pour donner un macrolide de formule (I) dans laquelle R représente Cl, Br ou I, ou

(v) un mercaptan de formule $HSR^5$, pour donner un macrolide de formule (I) dans laquelle R représente $SR^5$, ou

(vi) un acide carboxylique ou sulfonique de formule ArOH où Ar représente un groupe Ar de la catégorie (iii) pour donner un macrolide de formule (I) dans laquelle R représente OAr;

(d) faire réagir un macrolide de départ de formule (II) dans laquelle Q représente —$CH_2OH$ et $Q^1$ représente $R^1$, avec de la triphénylphosphine et une source d'halogène, pour donner un macrolide de formule (I) dans laquelle R représente Cl, Br ou I;

(e) faire réagir une matière de départ de formule (II) dans laquelle Q représente —$CH_2OH$ et $Q^1$ représente $R^1$, avec un agent d'acylation dérivant d'un acide carboxylique ou sulfonique de formule ArOH où Ar représente un groupe Ar de la catégorie (iii), pour donner un macrolide de formule (I) dans laquelle R représente OAr; ou

(f) faire réagir un macrolide de départ de formule (II) dans laquelle Q représente —$CH_2L$ où L est un groupe qui s'éloigne, et $Q^1$ représente $R^1$ ou un groupe qui s'éloigne, avec

(i) un halogénure ou un azide d'un métal alcalin ou encore un fluorure ou un azide de tétra-alkylammonium où le groupe alkyle est un groupe méthyle, éthyle, propyle ou butyle, pour donner un macrolide de formule (I) où R représente un groupe azido, F, Cl, Br ou I, ou

(ii) un ion mercaptide de formule $R^5S$—; pour donner un macrolide de formule (I) dans laquelle R représente $R^5S$—, ou

(iii) une amine de formule $NR^6R^6$ ou $HR^9$, pour donner un macrolide de formule (I) dans laquelle R représente $NR^6R^6$ ou $R^9$, ou

(iv) une source d'ion cyanure, pour donner un macrolide de formule (I) dans laquelle R représente —CN;

(v) un alcool de formule $HOR^4$ et une source d'ion argent pour donner un macrolide de formule (I) dans laquelle R représente $OR^4$ où $R^4$ est différent de l'hydrogène;

(g) hydrolyser un macrolide de formule (II) dans laquelle Q représente $CH_2R$ et $Q^1$ représente

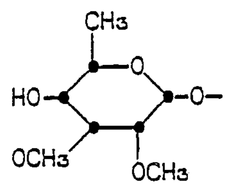 , 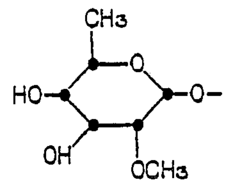 , ou 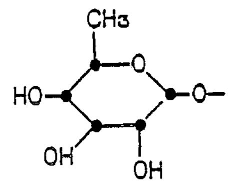

pour donner un macrolide de formule (I) dans laquelle $R^1$ représente un groupe hydroxyle; ou

(h) éliminer le groupe protecteur du groupe hydroxyle, d'un macrolide de formule (II) dans laquelle Q représente $—CH_2R$ et $Q^1$ représente un groupe hydroxyle protégé; ou

(i) faire réagir un macrolide de départ de formule (II) dans laquelle Q représente $—CH_2R$ et $Q^1$ représente un groupe hydroxyle, avec un azodicarboxylate de diéthyle ou de diméthyle, de la triphénylphosphine et un réactif choisi parmi

(i) un agent de transfert d'azide, pour donner un macrolide de formule (I) dans laquelle $R^1$ représente un groupe azido,

(ii) un phtalimide, pour donner un macrolide de formule (I) dans laquelle $R^1$ représente un groupe phtalimido,

(iii) un phénol de formule ArOH où Ar représente un groupe Ar de la catégorie (i) ou (ii), pour donner un macrolide de formule (I) dans laquelle $R^1$ représente —OAr,

(iv) un halogénure ou un polyhalogénure d'alkyle, pour donner un macrolide de formule (I) dans laquelle $R^1$ représente Cl, Br ou I, ou

(v) un mercaptan de formule $HSR^5$, pour donner un macrolide de formule (I) dans laquelle R représente $SR^5$, ou

(vi) un acide carboxylique ou sulfonique de formule ArOH, où Ar représente un groupe Ar de la catégorie (i), pour donner un macrolide de formule (I) où $R^1$ représente OAr; ou

(j) faire réagir un macrolide de formule (II) dans laquelle Q représente $—CH_2R$ et $Q^1$ représente un groupe hydroxyle, avec un agent d'acylation dérivant d'un acide carboxylique ou sulfonique de formule ArOH où Ar est un groupe de la catégorie (iii), pour donner un macrolide de formule (I) dans laquelle $R^1$ représente OAr; ou

(k) faire réagir un macrolide de formule (II) dans laquelle Q représente R et $Q^1$ représente un groupe hydroxyle, avec de la triphénylphosphine et un agent d'halogénation, pour donner un macrol' de formule (I) dans laquelle $R^1$ représente Cl, Br ou I; ou

(l) faire réagir un macrolide de formule (II) dans laquelle Q représente $—CH_2R$ et $Q^1$ représente un groupe qui s'éloigne, avec

(i) un halogénure ou un azide d'un métal alcalin ou encore un fluorure ou un azide de tétra-alkylammonium ou le groupe alkyle est un groupe méthyle, éthyle, propyle ou benzyle, pour donner un macrolide de formule (I) dans laquelle $R^1$ représente un groupe azido, F, Cl, Br ou I,

(ii) un ion mercaptide de formule $R^5S—$ pour donner un macrolide de formule (I) dans laquelle $R^1$ représente $R^5S—$, ou

(iii) une amine de formule $HNR^6R^6$ ou $HR^9$, pour donner un macrolide de formule (i) dans laquelle $R^1$ représente $HR^6R^6$ ou $R^9$; ou

(m) réduire un macrolide de formule (II) dans laquelle Q représente $—CH_2N_3$ et $Q^1$ représente $R^1$ ou Q représente R et $Q^1$ représente un groupe azido, pour donner un macrolide de formule (I) dans laquelle R représente $—CH_2NH_2$ ou $R^1$ représente un groupe amino; ou

(n) acyler un macrolide de formule (II) dans laquelle Q représente $—CH_2NHR^6$ ou $R^1$ représente $NHR^6$, pour donner un macrolide de formule (I) dans laquelle R représente $—CH_2NHR^7$ ou $R^1$ représente $—NR^6R^7$; ou

(o) estérifier un macrolide de formule (I); ou

(p) salifier un macrolide de formule (I); ou

(q) hydrolyser un macrolide de formule (I) dans laquelle $R^3$ est un groupe mycarosyloxy en solution acide, à un pH inférieur à 4, pour donner un macrolide de formule (I) dans laquelle $R^3$ représente un groupe hydroxyle; ou

(r) désoxygéner un macrolide de formule (I) dans laquelle $R^3$ représente un groupe hydroxyle, pour donner un macrolide de formule (I) dans laquelle $R^3$ représente un atome d'hydrogène;

(s) faire réagir un macrolide de formule (II) dans laquelle Q représente $CH_2I$ et $Q^1$ représente $R^1$, avec un agent réducteur, pour donner un composé de formule (I) dans laquelle R représente un atome d'hydrogène; ou

(t) faire réagir un macrolide de formule (II) dans laquelle Q représente un groupe $—CH_2—$ sulfonate et $Q^1$ représente $R^1$, avec une source d'ion iodure, pour donner un macrolide de formule (I) dans laquelle R représente I.

6. Macrolide de formule (I) ou un de ses sels physiologiquement acceptables selon l'une quelconque des revendications 1 à 5, caractérisé en ce qu'on l'utilise comme antibiotique dans la chimiothérapie des animaux à sang chaud.

7. Prémélange de nourriture, caractérisé en ce qu'il comprend, comme ingrédient actif, un macrolide de formule (I) ou un de ses sels physiologiquement acceptables selon l'une quelconque des revendications 1 à 5.

8. Formulation vétérinaire, caractérisée en ce qu'elle comprend, comme ingrédient actif, un macrolide de formule (I) ou un de ses sels physiologiquement acceptables selon l'une quelconque des revendications 1 à 5, en association avec un ou plusieurs supports ou véhicules physiologiquement acceptables pour ce macrolide ou ce sel.

**Revendications pour l'Etat contractant: AT**

1. Procéde de préparation d'un macrolide de formule (I):

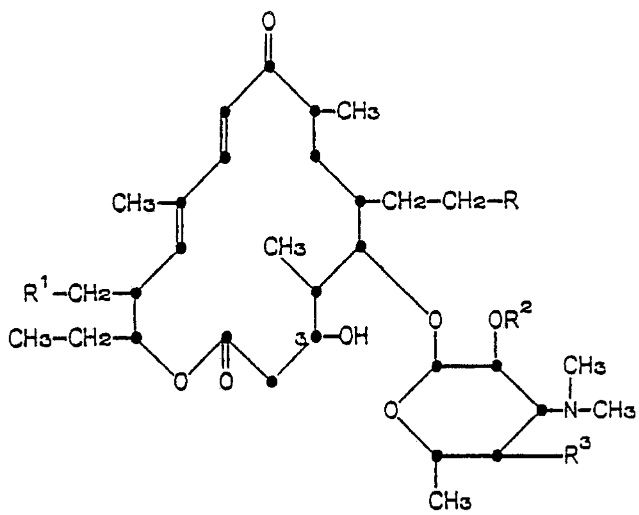

(I)

dans laquelle

R représente un atome d'hydrogène, d'iode, de brome, de chlore ou de fluor, un groupe cyano, $—OR^4$, —OAr, $—SR^5$, azido, $—NR^6R^7$, N-phtalimido ou $R^9$,

$R^1$ représente i) un atome d'hydrogène ou —OH; ii) un atome de chlore, de fluor, de brome, d'iode, un groupe —OAr, —O-tétrahydrofurannyle, —O-tétrahydropyrannyle, $—SR^5$, azido, $—NR^6R^7$ ou N-phtalimido; $R^9$;

$R^9$ représente i) un groupe amino monocyclique de formule $—N(CH_2)_n$ facultativement substitué sur un ou plusieurs atomes de carbone par un groupe alkyle en $C_1—C_3$, hydroxyle, méthoxy, éthoxy, $—N(R^8)_2$,

$$—\overset{\overset{O}{\|}}{C}—N(R^8)_2,$$

carbométhoxy, carbéthoxy ou phényle; et n est un nombre entier de 4 à 15; ii) un noyau hétérocyclique azoté monocyclique saturé ou non relié par l'atome d'azote, ce noyau comportant 1) 5 à 7 atomes comprenant jusqu'à 3 hétéro-atomes supplémentaires choisis parmi l'azote, l'oxygène et le soufre et 2) jusqu'à 3 substituants choisis parmi le groupe méthyle, éthyle, et phényle; ou iii) un groupe sec.-amino bicyclique ou tricyclique choisi parmi les groupes 1,2,3,4-tétrahydro-quinoléin-1-yle; décahydro-quinoléin-1-yle; 1,2,3,4-tétrahydro-isoquinoléin-2-yle; décahydroiso-quinoléin-2-yle; indolin-1-yle; isoindolin-2-yle; décahydrocyclohepta[b]-pyrrol-1-yle; décahydrocyclohepta[c]-pyrrol-2-yle; décahydrocyclopent[c]-azépin-2-yle; décahydrocyclopent[d]-azépin-3-yle; 2,3,4,5-tétrahydro-1H-2-benzazépin-2-yle; 2,3,4,5-tétrahydro-1H-3-benzazépin-3-yle; azabicycloheptanyle; azabicyclooctanyle; azabicyclononanyle; azabicyclodécanyle ou azatricyclodécanyle;

$R^2$ représente un atome d'hydrogène, un groupe alcanoyle en $C_1—C_5$ facultativement substitué ou un groupe phényl-propionyle, phénylacétyle ou benzoyle facultativement substitué;

$R^3$ représente un atome d'hydrogène, un groupe hydroxyle, un groupe alcanoyl(en $C_1—C_5$)oxy facultativement substitué ou un groupe phényl-propionyloxy, phénylacétoxy, benzoyloxy, benzoyloxy facultativement substitué ou

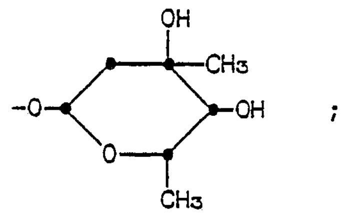

(mycarosyloxy)

$R^4$ représente un atome d'hydrogène, un groupe alkyle en $C_1$—$C_4$ facultativement substitué, un groupe cyclohexyle, un groupe phénoxyéthyle, phénéthyle ou benzyle facultativement substitué;

Ar représente i) un groupe phényle, un groupe phényle à dérivation ou un groupe naphtyle; ii) un groupe hétéroaryle facultativement substitué choisi parmi les groupes pyridinyle, pyrimidinyle, pyridazinyle, pyrazinyle, triazinyle, indolyle, isoquinoléinyle, quinoléinyle, quinazolinyle, cinnolinyle, quinoxalinyle, phtalazinyle, benzotriazolyle, benzoxazolyle, benzimidazolyle, carbazolyle ou acridinyle; ou iii) un groupe alcanoyle en $C_1$—$C_5$ facultativement substitué; un groupe phénylthio-acétyle, phénoxyacétyle, phénylpropionyle, phénylacétyle ou benzoyle facultativement substitué; un groupe méthanesulfonyle; un groupe trifluorométhanesulfonyle; ou un groupe phénylsulfonyle facultativement substitué;

$R^5$ représente un groupe alkyle en $C_1$—$C_4$ facultativement substitué; un groupe cyclohexyle; un groupe phénéthyle, benzyle ou phényle facultativement substitué; ou groupe hétéroaryle facultativement substitué et choisi parmi les groupes imidazolyle, pyrazolyle, pyridinyle, pyrimidinyle, pyrazinyle, pyridazinyle, triazinyle, triazolyle, tétrazolyle, oxazolyle, isoxazolyle, oxadiazolyle, thiazolyle, isothiazolyle, thiadiazolyle, thiényle et furannyle;

$R^6$ representé un atome d'hydrogène, un groupe cycloalkyle en $C_3$—$C_8$, un groupe phénéthyle, un groupe benzyle, un groupe phényle ou un groupe alkyle en $C_1$—$C_6$ facultativement substitué;

$R^7$ représente un groupe $R^6$ ou un groupe alcanoyle en $C_1$—$C_5$ facultativement substitué, un groupe phénylthioacétyle, un groupe phénoxyacétyle, un groupe phénylpropionyle, un groupe phénylacétyle ou un groupe benzoyle facultativement substitué, ou encore un groupe alcoxycarbonyle; et

$R^8$ représente un atome d'hydrogène, un groupe méthyle, un groupe éthyle, un groupe n-propyle, ou un groupe isopropyle ou encore les groupes $R^8$ pris ensemble forment une fraction polyméthylène de telle sorte que —$N(R^8)_2$ constitue un groupe amino cyclique choisi parmi le groupe pyrrolidinyle, le groupe pipéridinyle, le groupe hexahydroazépinyle ou le groupe octahydroazocinyle;

avec cette réserve que 1) lorsque $R^4$ représente un atome d'hydrogène, $R^1$ ne peut être un atome d'hydrogène ni un groupe OH; 2) lorsque R ou $R^1$ représente —$NHR^6$ ou que $R^4$ ou $R^8$ représente un atome d'hydrogène, $R^2$ doit être un atome d'hydrogène, $R^3$ doit également être un atome d'hydrogène, un groupe hydroxyle ou un groupe mycarosyloxy et Ar ne peut être un substituant de type (iii); 3) lorsque $R^2$ représente un atome d'hydrogène, $R^3$ doit être un atome d'hydrogène, un groupe hydroxyle ou un groupe mycarosyloxy; 4) au moins un des radicaux R et $R^1$ représente $R^4$; 5) lorsque $R^1$ représente H ou OH, $R^9$ ne représente pas un noyau hétérocyclique azoté insaturé ou saturé monocyclique comme défini sub ii); ou d'un sel physiologiquement acceptable de ce macrolide, caractérisé en ce q'il comprend les étapes qui consistent à:

(a) réduire un macrolide de départ de formule (II) dans laquelle Q représente le groupe formyle et $Q^1$ représente $R^1$, avec cette réserve que $R^1$ ne représente pas un groupe hydroxyle, pour obtenir un macrolide de formule (I) dans laquelle R représente un groupe hydroxyle;

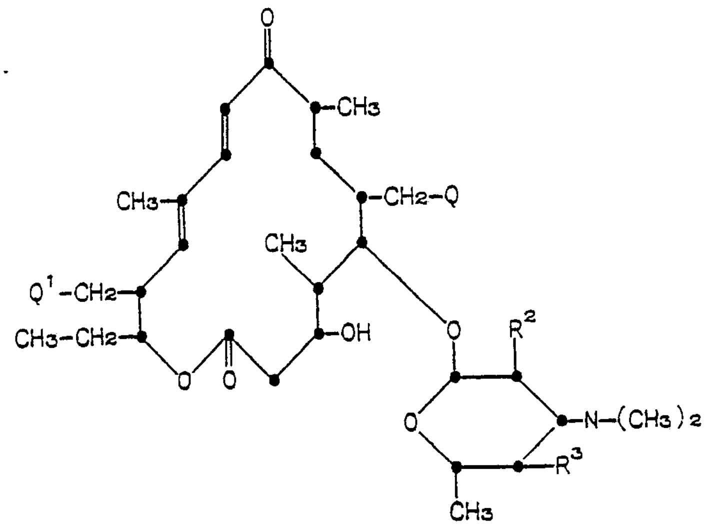

(II)

(b) faire réagir un macrolide de départ de formule (II) dans laquelle Q représente un groupe formyle, tandis que $Q^1$ représente $R^1$, avec une amine de formule $HNR^6R^6$ ou $HR^9$ en présence d'un agent réducteur pour obtenir un macrolide de formule (I) dans laquelle R représente $NR^6R^6$ ou $R^9$; ou

(c) faire réagir un macrolide de départ de formule (II) dans laquelle Q représente $—CH_2OH$ et $Q^1$ représente $R^1$, avec de l'azodicarboxylate de diéthyle ou de l'azodicarboxylate de diméthyle, la triphénylphosphine et un réactif choisi parmi

(i) un agent de transfert d'azide pour obtenir un macrolide de formule (I) dans laquelle R représente un groupe azido,

(ii) un phtalimide pour obtenir un macrolide de formule (I) dans laquelle R représente un groupe phtalimido,

(iii) un phénol de formule ArOH pour obtenir un macrolide de formule (I) dans laquelle R représente —OAr, où Ar représente un groupe Ar de la catégorie i) ou ii),

(iv) un halogénure ou un polyhalogénure d'alkyle pour obtenir un macrolide de formule (I) dans laquelle R représente Cl, Br ou I, ou

(v) un mercaptan de formule $HSR^5$, pour obtenir un macrolide de formule (I) dans laquelle R représente $SR^5$, ou

(vi) un acide carboxylique ou sulfonique de formule ArOH dans laquelle Ar est un groupe Ar de la catégorie (iii) pour obtenir un macrolide de formule (I) dans laquelle R représente OAr;

(d) faire réagir un macrolide de départ de formule (II) dans laquelle Q représente $—CH_2OH$ et $Q^1$ représente $R^1$, avec de la triphénylphosphine et une source d'halogène, pour obtenir un macrolide de formule (I) dans laquelle R représente Cl, Br ou I;

(e) faire réagir une matière de départ de formule (II) dans laquelle Q représente $—CH_2OH$ et $Q^1$ représente $R^1$, avec un agent d'acylation dérivant d'un acide carboxylique ou sulfonique de formule ArOH dans laquelle Ar représente un groupe Ar de la catégorie (iii), pour donner un macrolide de formule (I) dans laquelle R représente OAr; ou

(f) faire réagir un macrolide de départ de formule (II) dans laquelle Q représente $—CH_2L$ où L représente un groupe qui s'éloigne et $Q^1$ représente $R^1$ ou un groupe qui s'éloigne, avec

(i) un halogènure ou un azide d'un métal alcalin, ou encore un fluorure ou un azide de tétra-alkylammonium où le groupe alkyle est un groupe méthyle, un groupe éthyle, un groupe propyle ou un groupe butyle, pour obtenir un macrolide de formule (I) dans laquelle R représente un groupe azido, F, Cl, Br ou I, ou

(ii) un ion mercaptide de formule $R^5S$ pour obtenir un macrolide de formule (I) dans laquelle R représente $R^5S—$, ou

(iii) une amine de formule $NR^6R^6$ ou $HR^9$ pour obtenir un macrolide de formule (I) dans laquelle R représente $NR^6R^6$ ou $R^9$, ou

(iv) une source d'ion cyanure pour obtenir un macrolide de formule (I) dans laquelle R représente —CN;

(v) un alcool de formule $HOR^4$ et une source d'ion argent pour obtenir un macrolide de formule (I) dans laquelle R représente $OR^4$ où $R^4$ est différent de l'hydrogène;

(g) hydrolyser un macrolide de formule (II) dans laquelle Q représente $CH_2R$ et $Q^1$ représente

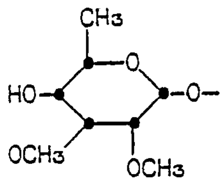 , 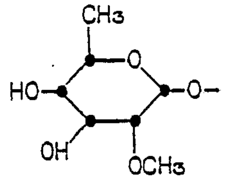 , ou 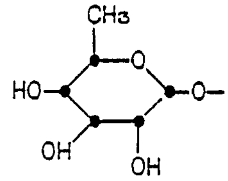

pour obtenir un macrolide de formule (I) dans laquelle $R^1$ représente un groupe hydroxyle; ou

(h) éliminer le groupe protecteur du groupe hydroxyle d'un macrolide de formule (II) dans laquelle Q représente $—CH_2R$ et $Q^1$ représente un groupe hydroxyle protégé, ou

(i) faire réagir un macrolide de départ de formule (II) dans laquelle Q représente $—CH_2R$ et $Q^1$ représente un groupe hydroxyle, avec un azodicarboxylate de diéthyle ou un azodicarboxylate de diméthyle, la triphénylphosphine, et un réactif choisi parmi

(i) un agent de transfert d'azide pour obtenir un macrolide de formule (I) dans laquelle $R^1$ représente un groupe azido,

(ii) un phtalimide, pour obtenir un macrolide de formule (I) dans laquelle $R^1$ représente un groupe phtalimido,

(iii) un phénol de formule ArOH dans laquelle Ar représente un groupe Ar de la catégorie (i) ou (ii), pour obtenir un macrolide de formule (I) dans laquelle $R^1$ représente —OAr,

(iv) un halogénure ou un polyhalogénure d'alkyle pour obtenir un macrolide de formule (I) dans laquelle $R^1$ représente Cl, Br ou I, ou

(v) un mercaptan de formule $HSR^5$ pour obtenir un macrolide de formule (I) dans laquelle R représente $SR^5$, ou

(vi) un acide carboxylique ou sulfonique de formule ArOH dans laquelle Ar est un groupe Ar de la catégorie (i), pour obtenir un macrolide de formule (I) dans laquelle $R^1$ représente un groupe OAr; ou

(j) faire réagir un macrolide de formule (II) dans laquelle Q représente $—CH_2R$ et $Q^1$ représente un groupe hydroxyle, avec un agent d'acylation dérivant d'un acide carboxylique ou sulfonique de formule ArOH dans laquelle Ar représente un groupe Ar de la catégorie (iii), pour obtenir un macrolide de formule (I) dans laquelle $R^1$ représente OAr; ou

(k) faire réagir un macrolide de formule (II) dans laquelle Q représente R et $Q^1$ représente un groupe hydroxyle, avec la triphénylphosphine et un agent d'halogénation, pour obtenir un macrolide de formule (I) dans laquelle $R^1$ représente Cl, Br ou I; ou

(l) faire réagir un macrolide de formule (II) dans laquelle Q représente $—CH_2R$ et $Q^1$ représente un groupe qui s'éloigne, avec

(i) un halogénure ou un azide d'un métal alcalin, ou encore un fluorure ou un azide de tétra-alkylammonium dans lequel le groupe alkyle est un groupe méthyle, un groupe éthyle, un groupe propyle ou un groupe benzyle, pour obtenir un macrolide de formule (I) dans laquelle $R^1$ est un groupe azido, F, Cl, Br ou I,

(ii) un ion mercaptide de formule $R^5S—$ pour obtenir un macrolide de formule (I) dans laquelle $R^1$ représente $R^5S—$, ou

(iii) une amine de formule $HNR^6R^6$ ou $HR^9$ pour obtenir un macrolide de formule (I) dans laquelle $R^1$ représente $HR^6R^6$ ou $R^9$, ou

(m) réduire un macrolide de formule (II) dans laquelle Q représente $—CH_2N_3$ et $Q^1$ représente $R^1$ ou Q représente R et $Q^1$ représente un groupe azido pour obtenir un macrolide de formule (I) dans laquelle R représente $—CH_2NH_2$ ou $R^1$ représente un groupe amino, ou

(n) acyler un macrolide de formule (II) dans laquelle Q représente $—CH_2NHR^6$ ou $R^1$ représente $NHR^6$ pour obtenir un macrolide de formule (I) dans laquelle R représente $—CH_2NHR^7$ ou $R^1$ représente $—NR^6R^7$, ou

(o) estérifier un macrolide de formule (I), ou

(p) salifier un macrolide de formule (I), ou

(q) hydrolyser un macrolide de formule (I) dans laquelle $R^3$ est un groupe mycarosyloxy, en solution acide à un pH inférieur à 4 pour obtenir un macrolide de formule (I) dans laquelle $R^3$ représente un groupe hydroxyle; ou

(r) désoxygéner un macrolide de formule (I) dans laquelle $R^3$ représente un groupe hydroxyle pour obtenir un macrolide de formule (I) dans laquelle $R^3$ représente un atome d'hydrogène;

(s) faire réagir un macrolide de formule (II) dans laquelle Q représente $CH_2I$ et $Q^1$ représente $R^1$, avec un agent réducteur pour obtenir un composé de formule (I) dans laquelle R représente un atome d'hydrogène, ou

(t) faire réagir un macrolide de formule (II) dans laquelle Q représente un groupe $—CH_2—$ sulfonate et $Q^1$ représente $R^1$, avec une source d'ion iodure pour obtenir un macrolide de formule (I) dans laquelle R représente un atome d'iode.

2. Procédé selon la revendication 1, en vue de préparer un macrolide de formule (I) dans laquelle R représente $R^9$.

3. Procédé selon la revendication 1, en vue de préparer un macrolide de formule (I) dans laquelle R

représente un atome d'hydrogène, un groupe hydroxyle, un groupe phénoxy, un groupe N-phtalimido, un groupe phénylacétoxy, un groupe 3-azabicyclo(3,2,2)-nonan-3-yle, un groupe morpholino, un groupe 4-phénylpipéridin-1-yle ou un groupe octahydroazocin-1-yle.

4. Procédé selon la revendication 1, en vue de préparer un macrolide de formule (I) dans laquelle $R^1$ représente un atome d'hydrogène, un groupe hydroxyle, un groupe phénoxy, un groupe acétoxy, un groupe phénylacétoxy, un groupe N-phtalimido, un groupe phénylpropionyloxy, un groupe phénylthio, un groupe octahydroazocin-1-yle, un groupe 4-hydroxypipéridino, un groupe 2,3-diméthoxyphénoxy, un groupe 3-pyridyloxy ou un groupe m-diméthylaminophénoxy.

5. Macrolide de formule (I) ou un de ses sels physiologiquement acceptables comme défini dans l'une quelconque des revendications 1 à 4, en vue de l'utiliser pour la suppression ou le traitement des infections bactériennes ou mycoplasmiques chez les animaux à sang chaud n'appartenant pas au règne humain.